# EUROPEAN PATENT SPECIFICATION

(11) **EP 4 395 750 B1**
(45) Date of publication and mention of the grant of the patent: **28.01.2026**
(21) Application number: 22865059.4
(22) Date of filing: 01.09.2022
(51) Int. Cl.: A61K 9/70, A61K 31/13, A61P 25/28, C07C 211/38, C07C 53/126

(54) **PERCUTANEOUS ABSORPTION PREPARATION FOR TREATING DEMENTIA COMPRISING MEMANTINE ENANTHATE**
ZUBEREITUNG ZUR PERKUTANEN ABSORPTION ZUR BEHANDLUNG VON DEMENZ MIT MEMANTINENANTHAT
PRÉPARATION D'ABSORPTION PERCUTANÉE POUR LE TRAITEMENT DE LA DÉMENCE COMPRENANT DE LA MÉMANTINE ÉNANTHATE

(30) Priority: 02.09.2021 KR 20210116832
(43) Date of publication of application: 10.07.2024
(73) Proprietor: Dong-A ST Co., Ltd., Seoul 02587 (KR)
(72) Inventor: JANG, Sun-Woo, Seoul 04358 (KR); SHIN, Chang-Yell, Seoul 05070 (KR); KIM, Hae-Sun, Hwaseong-si Gyeonggi-do 18486 (KR); HYUN, Sang-Min, Yongin-si Gyeonggi-do 17074 (KR); KIM, Yong-Jik, Yongin-si Gyeonggi-do 17073 (KR); SHIN, Chang-Yong, Yongin-si Gyeonggi-do 16809 (KR); KIM, Jae-Han, Yongin-si Gyeonggi-do 17073 (KR)
(74) Representative: Kraus & Lederer PartGmbB
(86) International application number: PCT/KR2022/013084
(87) International publication number: WO 2023/033557

(56) References cited:
- WO-A1-2012/051333
- WO-A1-2014/199455
- WO-A1-2018/199018
- JP-A- 2009 013 171
- KR-A- 20190 078 893
- US-A1- 2011 059 141
- US-A1- 2020 261 442

## Description

### Technical Field

The present invention relates to an improved pharmaceutical salt of memantine and a percutaneous absorption preparation for the treatment of dementia comprising the same, which has outstanding skin permeability, is continuously released for a long time, and causes minimal skin irritation.

### Background Art

Alzheimer's disease is caused by a neurodegenerative process with loss of cells in a cortical region or brain area. It is known to be caused by a decrease in acetylcholine, a neurotransmitter in the brain, or excessive activation of glutamate, another neurotransmitter, which induces neuronal death. Glutamate is a neurotransmitter that plays a very important role in memory and learning. Stimulation of N-methyl-D-aspartic acid (NMDA) receptors leads to excess calcium flows into neurons, leading to apoptosis in which cell components such as the cytoskeleton, cell membrane, and DNA are broken down. This is known to cause neurodegeneration in the long term.

Memantine is an NMDA receptor antagonist and is commercially available as Ebixa^{®}, an oral tablet approved by the US Food and Drug Administration in 2003. It has been reported that memantine's NMDA receptor blockade prevents nerve cell damage that may occur in dementia patients and improves symptoms by restoring the physiological function of the remaining nerve cells. It shows efficacy in patients with moderate or severe, rather than mild, Alzheimer's disease, and when there is no apparent improvement in symptoms even with acetylcholinesterase inhibitors, it is administered alone or in combination with acetylcholinesterase inhibitors to moderate or more severe Alzheimer's disease patients. The initial dose of memantine is 5 mg: 5 mg per day for the first week, 10 mg per day for the second week (1/2 tablet, twice a day), and 15 mg per day for the third week (1 tablet in the morning, 1/2 in the evening), and from the 4th week onwards, 20 mg per day as a maintenance dose, and one 10 mg tablet twice a day. Taking memantine oral tablets causes side effects such as dizziness, headache, constipation, drowsiness, increased blood pressure, and difficulty with breathing due to a rapid increase in blood concentration. The oral tablet form can also present challenges or inconvenience for elderly patients with impaired swallowing ability.

The memantine oral formulations currently in the market are in the form of a watersoluble hydrochloride, which is difficult to dissolve in solvent-based adhesives such as acrylic adhesives and rubber adhesives used in percutaneous absorption formulations. Memantine hydrochloride also crystallizes over time, causing precipitation, which leads to reduced skin permeability and reduced adhesive strength. Studies have been conducted on percutaneous absorption preparations of memantine free base instead of memantine hydrochloride. In the case of memantine free base, log P, the partition coefficient of the drug, is 3.28. It penetrates the skin easily due to its strong lipophilicity, but it causes severe skin irritation. It also has a melting point of 10°C or lower and is highly volatile, which may cause a decrease in the amount of the effective drug and stability problems during the preparation and storage of the percutaneous absorption preparation.

To solve the above-mentioned problems of memantine-containing drugs including difficulty with oral administration and the side effects caused by the physicochemical properties of memantine salt, researchers around the world have been working on percutaneously absorbable preparation for dementia treatment comprising memantine or a salt thereof. However, there is no memantine-containing percutaneously absorbable preparation on the market yet due to the low skin permeability, skin irritation, and the problems of physicochemical stability such as crystal precipitation.

Various efforts made to overcome the low skin permeability and skin irritation of memantine-containing percutaneous absorption preparations can be found in US Patent Application Publication No. 2019-0183810, Korean Patent Application Publication No. 2019-0032551, Korean Patent No. 10-1964295, US Patent No. 8,882,729, and International Patent Application Publication No. WO 2014-174564.

US Patent Application Publication No. 2019-0183810 relates to a percutaneous absorption preparation comprising memantine free base, which is volatile or has a low melting point in an adhesive matrix, and glycolate, lactate, alpha hydroxybutyrate, pyruvate, acetoacetate, or levulinate as counteranion. It discloses a transdermal drug delivery system comprising multiple drug-containing layers to prevent loss of the main ingredient of the memantine free base and to achieve therapeutic blood concentrations of memantine for about 3-7 days. It does not teach sufficient skin permeability and safety, as the maximum skin permeability in human cadaver skin was 10 ug/cm2/hr at maximum.

Korean Patent Application Publication No. 2019-0032551 relates to a multi-layered transdermal drug delivery system comprising memantine base prepared by reacting memantine hydrochloride with an alkali salt such as sodium bicarbonate or potassium bicarbonate in a drug storage layer. It tried to prevent crystal precipitation due to low solubility, a problem that occurs when memantine hydrochloride oral formulation is applied as a transdermally absorbed formulation, and to improve low skin permeability. Although it achieved skin permeability at a consistent level of 15 ug/cm2/hr for 7 days in human cadaver skin, it does not teach sufficient skin permeability and safety of drug exposure and skin irritation.

Korean Patent No. 10-1964295 relates to a percutaneous absorption preparation comprising stearic acid salt, polymethacrylate, polyvinylpyrrolidone polymer with a molecular weight of 400,000-3,000,000, or a mixture thereof as a release control agent, in order to minimize skin irritation during transdermal administration by controlling the release rate of memantine free base for high skin permeation. It shows that erythema and edema due to human skin irritation are minimized when the skin absorption rate is less than 20 ug/cm2/h for 24 hours in human cadaver skin. However, it does not teach sustained skin penetration and the safety of the drug when the drug is applied for a long period of time.

U.S. Patent No. 8,882,729 relates to a transdermal therapeutic system comprising memantine or a physiologically acceptable salt. It discloses a multilayer transdermal drug delivery system comprising memantine free base produced by reacting memantine hydrochloride with an alkali salt such as calcium chloride and sodium ethanolate in the drug-containing layer. The prior art discloses that the plasma concentration of memantine can be maintained after 3 days of administration in a rabbit animal model. And through pharmacokinetic studies at the 24-hour administration interval in clinical trials, the minimum plasma concentration of 65 ng/mL or more was estimated after 300 hours. However, as there are no experimental data related to skin permeability disclosed in the prior art, it does not teach sustained skin penetration and safety of the drug when the drug is applied for a long period of time.

International Application Publication WO 2014-174564 relates to an adhesive patch drug formulation comprising memantine or a pharmaceutically acceptable salt thereof and an organic acid salt in a drug-containing layer. Examples of such organic acids include dicarboxylic acids such as oxalic acid and azelaic acid; hydroxycarboxylic acids such as glycolic acid and lactic acid; aromatic carboxylic acids such as benzoic acid and salicylic acid; caproic acid, caprylic acid, capric acid, undecenoic acid, lauric acid, myristic acid, palmitic acid, stearic acid, linoleic acid, oleic acid, and isostearic acid. The prior art discloses maintaining the content by inhibiting memantine crystallization and memantine volatilization during preparation or storage of the patch. However, as there are no experimental data related to skin permeability, it does not teach about continuous skin permeation and safety when a percutaneous absorption formulation is actually applied for a long term.

WO 2014-199455 discloses a patch preparation containing memantine or a pharmaceutically acceptable salt thereof, including memantine hydrochloride, the patch preparation being produced while inhibiting memantine volatilization to a sufficient level. Also JP 2009013171A discloses a transdermal preparation useful for treating Alzheimer type dementia and comprising memantine hydrochloride as active ingredient.

Against this background, the present inventors, in order to solve the existing problems of low skin permeability and strong skin irritation with percutaneous absorption formulations containing memantine, synthesized various novel pharmacologically acceptable salts for memantine free base. Through evaluation of solubility for solubilizers (absorption enhancers or skin penetration enhancers), skin permeability evaluation, crystallization evaluation, evaluation of skin irritation in hairless mice and rabbits, and pharmacokinetic evaluation, the present inventors found that, compared to existing percutaneous absorption formulations comprising memantine salt or other novel salts, a percutaneous absorption preparation comprising memantine enanthate exhibits high skin permeability without crystallization in various solubilizers, and that it is constantly released over a long period of time. Also, percutaneous absorption preparations comprising memantine free base show moderate or more skin irritation such as severe erythema and edema. The present inventors discovered that a percutaneous absorption formulation comprising memantine enanthate exhibits mild skin irritation when applied for 7 days, thereby improving skin irritation.

### Disclosure of Invention

### Technical Problem

An object of the present invention is to provide a percutaneous absorption preparation comprising a memantine salt that exhibits high skin permeability without drug crystal precipitation, has a constant release pattern for a long period of time, and has improved skin irritation safety.

### Solution to Problem

The present invention provides an enanthate salt of memantine.

The memantine used in the drug-containing layer of the present invention is an NMDA receptor antagonist that selectively binds to NMDA, a receptor for glutamate, an excitatory neurotransmitter in the brain, and protects against nerve damage in the brain. It is used in the form of memantine enanthate.

For use in percutaneous absorption formulation, the salt of memantine must meet the following four physicochemical criteria: (1) high solubility in solubilizers; (2) outstanding skin permeability; (3) minimal crystal precipitation; and (4) minimal skin irritation.

As can be seen from the following Examples and Experimental Examples, the memantine enanthate of the present invention has a unique combination of excellent formulation characteristics that makes the transdermal preparation of memantine particularly suitable. That is, the memantine enanthate of the present invention exhibited characteristics satisfying all of the above four conditions. On the other hand, none of the other various salts tested for comparison in the present invention satisfies all the above four criteria, and in particular, memantine hydrochloride, a commercially available product, exhibited low solubility and low skin permeability in the patch.

Therefore, memantine enanthate according to the present invention provides a unique combination of excellent solubility, excellent skin permeability, minimal crystal precipitation and minimal skin irritation, which is very suitable for the preparation of memantine-containing percutaneous absorption preparations.

Another embodiment of the present invention provides a percutaneous absorption preparation for the treatment of dementia, comprising a support layer, a drug-containing layer, and a release layer, wherein the drug-containing layer comprises memantine enanthate, a solubilizer and a pressure-sensitive adhesive.

The memantine enanthate in the drug-containing layer of the present invention may be contained in an amount of 1 to 20 wt%, preferably 1.5 to 15 wt%, and more preferably 2 to 10 wt%, based on the total weight of the drug-containing layer.

For the solubilizer of the present invention, a solubilizer having a solubility of 30 mg/ml or more with respect to memantine enanthate can be used. For example, propylene glycol monocaprylate, propylene glycol monolaurate, diisopropyl adipate, diisopropyl sebacate, oleic acid, isopropyl palmitate, glyceryl monocaprylate, isostearate, cocoyl caprylocaprate, cetyl 2-ethylhexanoate, oleoyl macrogol-6-glyceride, propylene glycol dicaprylocaprate, propylene glycol dicaprylocaprate, propylene glycol dicaprorate/dicaprate, glyceryl monooleate, 1-dodecyl - 2-pyrrolidinone, linoleoyl macrogol-6 glyceride, polyglyceryl-3 dioleate, ethyl oleate, isopropyl isostearate, isopropyl myristate, medium chain triglyceride, isocetyl myristate, isostearyl alcohol, oleyl alcohol, peanut oil, and diethylene glycol monoethyl ether have a solubility of 30 mg/ml or more in memantine enanthate. Preferably, one or more may be selected from the group consisting of propylene glycol monocaprylate, diisopropyl adipate, diisopropyl sebacate, isopropyl palmitate, isopropyl myristate, oleoyl macrogol-6-glyceride, isopropyl isostearate, and oleyl alcohol.

Such a solubilizer may be contained in the amount of 1-40 wt%, preferably 2-30 wt%, and more preferably 5-15 wt%, based on the total weight of the drug-containing layer, in consideration of the sufficient skin permeation enhancing effect of the percutaneous absorption preparation.

The pressure-sensitive adhesive of the present invention may be selected from the group consisting of a styrene-isoprene-styrene block copolymer (SIS), an acrylic polymer, and a polyisobutylene polymer.

The acrylic polymer used in the present invention is a polymer obtained by copolymerizing an acrylic ester with acrylic acid or derivatives thereof. Such as Duro-Tak 87-9301, Duro-Tak 87-2510, Duro-Tak 87-2287, Duro-Tak 87-4287, Duro-Tak 87-2516, Duro-Tak 87-235A, and Duro-Tak 87-2051, but is not limited thereto. The content of such an acrylic polymer is 50-90 wt%, preferably 55-85 wt%, more preferably 65-75 wt%, and even more preferably 70-80 wt%, based on the total weight of the drug-containing layer.

The polyisobutylene polymer used in the present invention is prepared by polymerizing isobutylene and may have a molecular weight in the range of 500 to 1,000,000 or more. The content of the polyisobutylene polymer is usually 10-90% by weight, preferably 20-80% by weight, more preferably 30-70% by weight, still more preferably 40-60% by weight, based on the total weight of the drug-containing layer.

A particularly preferable pressure-sensitive adhesive in the present invention is a styrene-isoprene-styrene block copolymer, which is a rubber-based adhesive. Styrene-isoprene-styrene block copolymer is a thermoplastic elastomer composed of styrene and isoprene. Its properties such as melting point and solution viscosity vary depending on the styrene content and diblock content in the styrene-isoprene-styrene block copolymer rubber.

The styrene-isoprene-styrene block copolymer used in the present invention is not particularly limited, but preferably, according to the "Method for measuring viscosity of styrene-isoprene-styrene block copolymer" described in the Japanese Pharmaceutical Additives Specifications 2013 Edition, when measuring the viscosity of the styrene block copolymer solution, the lower limit of the solution viscosity is 0.5 Pa*s or more, preferably 0.7 Pa*s or more, and more preferably 0.9 Pa*s or more; and although the upper limit of the solution viscosity is not particularly limited, it is preferably 2.0 Pa*s or less, and more preferably it is 1.8 Pa*s or less.

If the content of the pressure-sensitive adhesive in the drug-containing layer is too small, it is difficult to maintain the shape of the drug-containing layer. When the content of the pressure-sensitive adhesive in the drug-containing layer is too large, the skin permeability of the drug may be reduced. Therefore, the content of the styrene-isoprene-styrene block copolymer of the present invention is 10-70 wt%, more preferably 15-65 wt%, still more preferably 20-60 wt% or less, and particularly preferably 25-55 wt%, based on the total weight of the drug-containing layer.

A plasticizer may be contained in the adhesive composition of the percutaneous absorption preparation provided by the present invention. Plasticizers that can be used in the present invention include, but are not limited to, paraffinic process oil, naphthenic process oil, aromatic process oil, olive oil, camellia oil, tall oil, castor oil, isopropyl myristate, hexyl laurate, mineral oil, octyldodecyl myristate, propylene glycol, and propylene glycol monocaprylate. Two or more of these components may be mixed and used, and the content of the plasticizer, in consideration of the maintenance of sufficient cohesive strength of the percutaneous absorption preparation, is 10-80% by weight, preferably 20-75% by weight, more preferably 25-70% by weight, and still more preferably, 30-65% by weight, of the total weight of the drug-containing layer.

A tackifying resin may be added to the drug-containing layer of the present invention in order to adjust the adhesive strength of the percutaneous absorption preparation. Examples of the tackifying resin that can be used include, but are not limited to, a rosin derivative, an alicyclic saturated hydrocarbon resin, an aliphatic hydrocarbon resin, and the like. A terpene resin was used in the embodiments of the present invention, though it shall not be construed as limiting. If a tackifier is contained in the drug-containing layer, the content of the tackifier in the drug-containing layer is preferably 20 wt% or less, preferably 15 wt% or less, more preferably 10 wt% or less, still more preferably 8 wt% or less, of the total weight of the drug-containing layer, and most preferably, no tackifier is used at all, for reasons such as reducing skin irritation. That is, with respect to the skin adhesion of the patch, the content of the tackifier may be adjusted according to the type and mixing ratio of memantine enanthate, styrene-isoprene-styrene block copolymer, solubilizer, and plasticizer in the drug-containing layer. A tackifier is not required if there is sufficient skin adhesion without adding a tackifier.

In the present invention, the "support" in the support layer is not particularly limited, and one or more can be selected from those that are generally used for percutaneous absorption preparation, such as stretchable or non-stretchable woven or non-woven fabrics such as polyethylene, polypropylene, and polyethylene terephthalate; polyesters such as polyethylene terephthalate; polyolefins such as polyethylene and polypropylene; films such as polyurethane, ethylene vinyl acetate copolymer, and polyvinyl chloride; and foamable supports such as polyolefin and polyolethane; and may be used either alone or in combination stacked in layers. Also, in order to prevent static electricity from accumulating on the support, an antistatic agent may be contained in the electric woven fabric, nonwoven fabric, film, and the like constituting the support. Moreover, in order to obtain favorable anchoring with the adhesive layer, a nonwoven fabric, a woven fabric, or a laminate of a film can be used as a support body. The thickness of the support is usually 10-100 µm and preferably 15-50 µm for a film, and is usually 5-2,000 µm and preferably 100-1,000 µm for a porous sheet such as a woven fabric, non-woven fabric, or foam support.

For the release layer in the present invention, a general release liner generally used for percutaneous absorption preparations may be used. As the release liner, polyolefin such as glassine paper, polyethylene, and polypropylene; polyester such as polyethylene terephthalate; resin film such as polystyrene; aluminum film, foamed polyethylene film, or foamed polypropylene film, and the like can be used. Also, those processed with silicone or fluororesin, embossed, hydrophilic or hydrophobic processed, etc. can be used. The thickness of the release liner is usually 10-200 µm, and preferably 15-150 µm.

Furthermore, the percutaneous absorption preparation of the present invention may comprise additives, solubilizers, transdermal absorption accelerators, flavoring agents, coloring agents, and the like.

The percutaneous absorption of the present invention can be prepared by dissolving or dispersing an adhesive agent, memantine enanthate, a stabilizer, a plasticizer, and the like in a solvent; applying the resulting solution or dispersion onto the surface of the release layer; drying; and laminating support onto it. As an embodiment of the present invention, the memantine-containing percutaneous absorption preparation can be prepared by a method comprising the following steps: (1) dissolving a mixture of memantine enanthate and a solubilizer according to the present invention in an organic solvent; (2) applying the solution prepared in the above step onto the release layer and drying it, forming a drug-containing layer; and (3) laminating the drug-containing layer obtained in the above step with a support layer.

The solvent that can be used in the above-mentioned preparation method according to the present invention includes, for example, ethyl acetate, toluene, hexane, 2-propanol, methanol, ethanol, methylene chloride, and tetrahydrofuran. The temperature at the time of dissolution or dispersion of the adhesive and the like in the solvent is not particularly limited, but higher temperatures may increase the likelihood of solvent evaporation and may increase decomposition of memantine enanthate, causing formation of more impurities. As such, the preferred temperature range is at or below 80°C, and more preferably at or below 60°C.

Also, the method of applying the solution or dispersion to the release liner, the method of drying, and the method of laminating a support in the above-mentioned embodiment of the present invention can follow conventional methods of preparing percutaneous absorption preparations.

The percutaneous absorption preparation provided by the present invention may use an antioxidant if needed. As the antioxidant, a commonly known antioxidant or derivative thereof can be used, and examples thereof include ascorbic acid, ester derivatives thereof, sodium bisulfite, dibutylhydroxytoluene, butylhydroxyanisole, cysteine, glutathione, tryptophan, methionine, metasulfonic acid, malic acid, citric acid, benzotriazole, monothioglycerin, and the like, but are not limited thereto.

### Advantageous Effects of Invention

The percutaneous absorption preparation containing memantine enanthate according to the present invention exhibits high skin permeability without drug crystal precipitation, has a constant release pattern for a long period of time, and has improved safety by minimizing skin irritation.

Therefore, the percutaneous absorption preparation according to the present invention can be effectively used instead of the conventional oral preparation for the treatment of dementia.

### Brief Description of Drawings

Figure 1 shows the solubility of memantine enanthate and memantine levulinate depending on the type of solubilizer.
Figure 2 shows the skin permeability of the percutaneous absorption preparation according to the type of memantine salt.
Figure 3 shows the skin permeability according to the memantine salt type for the solubilizer diisopropyl sebacate.
Figure 4 shows the skin permeability according to the memantine salt type for the solubilizer diisopropyl adipate.
Figure 5 shows the skin permeability according to the memantine salt type for the solubilizer isopropyl palmitate.
Figure 6 shows the skin permeability according to the memantine salt type for the solubilizer isopropyl myristate.
Figure 7 shows the skin permeability according to the memantine salt type for the solubilizer oleoyl macrogol-6-glyceride.
Figure 8 shows the skin permeability according to the memantine salt type for the solubilizer isopropyl isostearate.
Figure 9 shows the skin permeability according to the memantine salt type for the solubilizer oleyl alcohol.
Figure 10 shows the skin permeability of the memantine enanthate percutaneous absorption preparation according to the reduction in the solubilizer and the change in the adhesive.
Figure 11 shows the evaluation of skin irritation in rabbits.

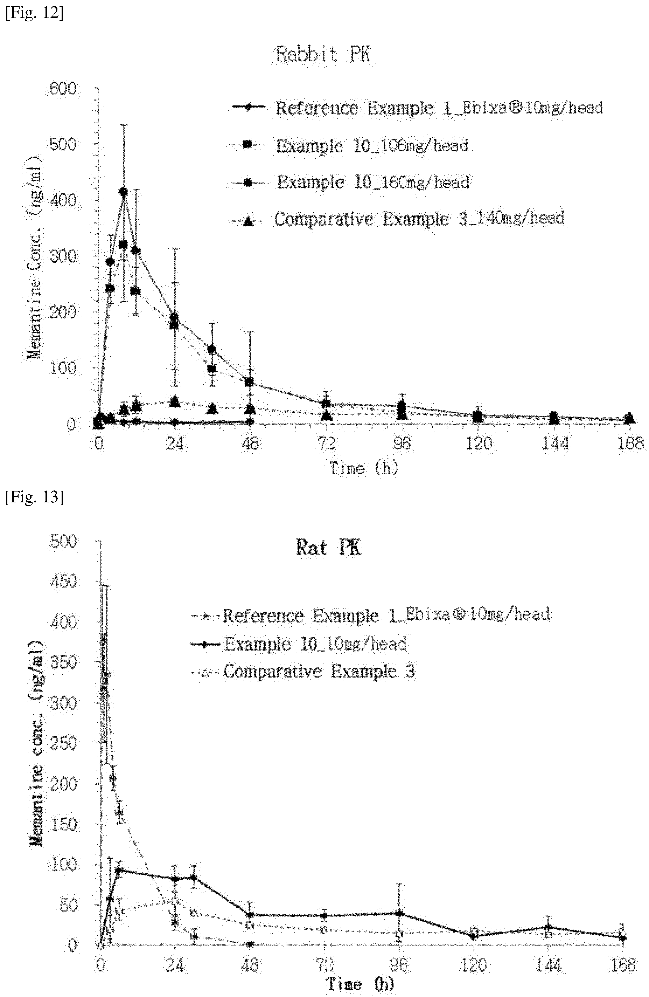

Figure 12 shows the blood concentration profile of percutaneous absorption preparation comprising memantine enanthate in rabbits.
Figure 13 shows the blood concentration profile of percutaneous absorption preparation comprising memantine enanthate in hairless mice.
Figure 14 shows the blood concentration profile according to the size and dose of percutaneous absorption preparation comprising memantine enanthate in hairless mice.
Figure 15 shows the maximum blood concentration and the area under the blood drug concentration-time curve according to the size and dose of percutaneous absorption preparation comprising memantine enanthate in hairless mice.

### Best Mode for Carrying out the Invention

The present invention is further described below with examples and experimental

### <Preparatory Example 1> Preparation of memantine salt

### 1. Preparation of memantine enanthate

Memantine free base (170.0 g) was added to the reaction unit, and acetonitrile (3.4 L) was added. Enanthic acid (124.1 g) was added, and the mixture was stirred at 60°C for 1 hour. The mixture was cooled to room temperature and then stirred overnight. The resulting solid was filtered, washed with acetonitrile, and dried under vacuum at room temperature overnight to obtain 269.5 g (91%) of the title compound.

1H NMR (400 MHz, DMSO-d6): 2.07 (m, 3H), 1.44 (t, 2H), 1.42 (s, 1H), 1.22 (m, 10H), 1.06 (d, 1H), 1.02 (d, 1H), 0.84 (t, 3H), 0.79 (s, 6H)

### 2. Preparation of memantine lactate - not according to the invention

Memantine free base (30.0 g) was added to the reaction unit, and isopropyl ether (600 mL) was added. Lactic acid (15.2 g) was added, and the mixture was stirred at 60°C for 1 hour. After stirring at room temperature for 3 hours, the resulting solid was filtered, washed with isopropyl ether, and vacuum dried overnight at room temperature to obtain 42.2 g (93%) of the title compound.

1H NMR (400 MHz, DMSO-d6): 3.57 (m, 1H), 2.11 (s, 1H), 1.56 (s, 2H), 1.38 (dd, 2H), 1.08 (m, 6H), 0.82 (s, 6H)

### 3. Preparation of memantine caprylate - not according to the invention

Memantine free base (3.0 g) was added to the reaction unit, and acetone (30 mL) was added. Caprylic acid (2.7 mL) was added, and the mixture was stirred at 60°C for 30 minutes. The mixture was cooled to room temperature and then stirred overnight. The resulting solid was filtered, washed with acetone, and dried in vacuo at room temperature overnight to obtain 4.6 g (85%) of the title compound.

1H NMR (600 MHz, DMSO-d6): 2.05 (s, 1H), 2.02 (t, 2H), 1.42-1.44 (m, 4H), 1.22-1.28 (m, 16H), 1.08 (dd, 1H), 1.02 (dd, 1H), 0.84 (t, 3H), 0.79 (s, 6H)

### 4. Preparation of memantine levulinate - not according to the invention

After memantine free base (180.0 g) was added to the reaction unit, acetone (450 mL) and ethyl acetate (450 mL) were added. After adding levulinic acid (117.2 g), the mixture was stirred at room temperature for 1 hour. The reaction product was concentrated under reduced pressure, and normal hexane (900 mL) was added to the concentrated residue. After stirring at room temperature for 3 hours, the solid was filtered, washed with n-hexane and vacuum dried overnight at room temperature to obtain 277.5 g (93%) of the title compound.

1H NMR (400 MHz, DMSO-d6): 2.49 (t, 2H), 2.18(t, 2H), 2.07 (s, 4H), 1.49 (s, 1H), 1.30 (d, 2H), 1.28 (s, 2H), 1.24 (d, 2H), 1.08 (d, 1H), 1.04 (d, 1H), 0.80 (s, 6H)

### 5. Preparation of memantine valerate - not according to the invention

After memantine free base (2.0 g) was added to the reaction unit, acetonitrile (40 mL) was added to dissolve it. After adding valeric acid (1.3 g), the mixture was stirred at room temperature for 5 hours. The resulting solid was filtered, washed with a small amount of acetonitrile, and vacuum dried at room temperature overnight to obtain 2.80 g (89%) of the title compound.

1H NMR (400 MHz, DMSO-d6): 2.05 (s, 1H), 2.00 (t, 2H), 1.41 (m, 2H), 1.28 (d, 2H), 1.25 (d, 2H), 1.21 (s, 2H), 1.07 (d, 1H), 1.03 (d, 1H), 0.83 (t, 3H), 0.79 (s, 6H)

### 6. Preparation of memantine xinafoate - not according to the invention

Memantine free base (2.0 g) was added to the reaction unit, and then methanol (20 mL) was added to dissolve it. After adding 1-hydroxy-2-naphthoic acid (2.3 g), the mixture was stirred at room temperature for 3 hours. After adding water (20 mL) to the reaction mixture, the mixture was further stirred for 2 hours. The resulting solid was filtered, washed with a small amount of water, and dried in vacuo at room temperature overnight to obtain 3.94 g (96%) of the title compound.

1H NMR (400 MHz, DMSO-d6): 8.16 (d, 1H), 7.69 (dd, 2H), 7.42 (t, 1H), 7.35 (t, 1H), 7.00 (d, 1H), 2.12 (s, 1H), 1.60 (s, 2H), 1.41 (d, 2H), 1.38 (d, 2H), 1.26 (s, 1H), 1.11 (d, 1H), 1.06 (d, 1H), 0.81 (s, 6H)

### 7. Preparation of memantine (+)-camphorsulfonate - not according to the invention

After memantine free base (2.0 g) was added to the reaction unit, acetone (10 mL) was added. After adding camphorsulfonic acid (2.9 g), the mixture was stirred at room temperature for 3 hours. The resulting solid was filtered, washed with acetone, and then vacuum dried at room temperature overnight to obtain 4.3 g (93%) of the title compound.

1H NMR (400 MHz, DMSO-d6): 7.44 (bs, 1H), 3.35 (s, 1H), 2.85 (d, 1H), 2.68 (m, 1H), 2.77 (d, 1H), 2.21 (dd, 1H), 2.15 (m, 1H), 1.93 (t, 1H), 1.85 (m, 1H), 1.80 (d, 1H), 1.59 (s, 2H), 1.40 (q, 4H), 1.28 (m, 6H), 1.56(d, 1H), 1.08 (d, 1H), 1.03 (s, 3H), 0.84 (s, 6H), 0.73 (s, 3H)

### 8. Preparation of memantine hemi-adipate - not according to the invention

Memantine free base (2.0 g) was added to the reaction unit, and then acetone (60 mL) was added to dissolve it. After adding adipic acid (0.82 g), the temperature was raised to 60°C, and it was stirred for 1 hour, cooled to room temperature, and stirred for 3 hours. The resulting solid was filtered, washed with acetone (10 mL), and then vacuum dried at room temperature overnight to obtain 2.75 g (76%) of the title compound.

1H NMR (400 MHz, DMSO-d6): 2.09 (m, 2H), 2.04 (s, 1H), 1.46 (s, 2H), 1.38 (s, 2H), 1.21 (s, 2H), 1.16 (d, 2H), 1.05 (d, 1H), 1.02 (d, 1H), 0.79 (s, 6H)

### 9. Preparation of memantine hemi-edisylate - not according to the invention

After memantine free base (2.0 g) was added to the reaction unit, acetonitrile (60 mL) was added to dissolve it. After adding edicylic acid (1.06 g), the temperature was raised to 60°C, and it was stirred for 1 hour, cooled to room temperature, and stirred for 3 hours. The resulting solid was filtered, washed with acetonitrile (10 mL), filtered, and then vacuum dried overnight at room temperature to obtain 2.50 g (82%) of the title compound.

1H NMR (400 MHz, DMSO-d6): 7.81 (s, 2H), 2.66 (s, 2H), 2.13 (s, 1H), 1.59 (s, 2H), 1.40 (d, 2H), 1.37 (d, 2H), 1.27 (s, 2H), 1.12 (d, 1H), 1.08 (d, 1H), 0.83 (s, 6H)

### 10. Preparation of memantine hemi-fumarate - not according to the invention

Memantine free base (2.0 g) was added to the reaction unit, and then acetone (60 mL) was added to dissolve it. After adding fumaric acid (0.65 g), the temperature was raised to 60°C, and it was stirred for 1 hour, cooled to room temperature, and stirred for 3 hours. The resulting solid was filtered, washed with acetone (10 mL), and then vacuum dried at room temperature overnight to obtain 2.58 g (98%) of the title compound.

1H NMR (400 MHz, DMSO-d6): 6.29 (s, 1H), 3.33 (s, 1H), 2.08 (s, 1H), 1.47 (s, 2H), 1.28 (d, 2H), 1.24 (s, 2H), 1.22 (d, 2H), 1.08 (d, 1H), 1.04 (d, 1H), 0.80 (s, 2H)

### 11. Preparation of memantine hemi-glutamate - not according to the invention

Memantine free base (2.0 g) was added to the reaction unit, and then acetone (60 mL) was added to dissolve it. After adding glutaric acid (0.74 g), the temperature was raised to 60°C, and it was stirred for 1 hour, cooled to room temperature, and stirred for 3 hours. The resulting solid was filtered, washed with acetone (10 mL), and then vacuum dried at room temperature overnight to obtain 2.71 g (99%) of the title compound.

1H NMR (400 MHz, DMSO-d6): 2.12 (d, 2H), 2.06 (s, 1H), 1.65 (m, 1H), 1.42 (s, 1H), 1.25 (d, 2 H), 1.24 (s, 2H), 1.22 (d, 2H), 1.07 (d, 1H), 1.03 (d, 1H), 0.80 (s, 6H)

### 12. Preparation of memantine hemi-malate - not according to the invention

Memantine free base (2.0 g) was added to the reaction unit, and then ethyl acetate (60 mL) was added to dissolve it. After adding L-malic acid (0.75 g), the temperature was raised to 60°C, and it was stirred for 1 hour, cooled to room temperature, and stirred for 3 hours. The resulting solid was filtered, washed with ethyl acetate (10 mL), and then vacuum dried at room temperature overnight to obtain 2.55 g (93%) of the title compound.

1H NMR (400 MHz, DMSO-d6): 3.80 (d, 0.5H), 2.43 (d, 0.5H), 2.21 (d, 0.5H), 2.08 (s, 1H), 1.47 (s, 2H), 1.29 (d, 2H), 1.24 (s, 2H), 1.22 (d, 2H), 1.11 (d, 1H), 1.09 (d, 1H), 0.81 (s, 6H)

### 13. Preparation of memantine hemi-maleate - not according to the invention

After memantine free base (2.0 g) was added to the reaction unit, acetonitrile (60 mL) was added to dissolve it. After adding maleic acid (0.65 g), the temperature was raised to 60°C, and it was stirred for 1 hour, cooled to room temperature, and stirred for 3 hours. The resulting solid was filtered, washed with acetonitrile (10 mL), and then vacuum dried at room temperature overnight to obtain 2.56 g (97%) of the title compound.

1H NMR (400 MHz, DMSO-d6): 6.01 (s, 1H), 2.08 (s, 1H), 1.44 (s, 2H), 1.26 (d, 2H), 1.24 (s, 2H), 1.22 (d, 2H), 1.10 (d, 1H), 1.04 (d, 1H), 0.80 (s, 6H)

### 14. Preparation of memantine hemi-malonate - not according to the invention

Memantine free base (2.0 g) was added to the reaction unit, and then acetone (60 mL) was added to dissolve it. After adding malonic acid (0.58 g), the temperature was raised to 60°C, and it was stirred for 1 hour, cooled to room temperature, and stirred for 3 hours. The resulting solid was filtered, washed with acetone (10 mL), and then vacuum dried at room temperature overnight to obtain 2.57 g (99%) of the title compound.

1H NMR (400 MHz, DMSO-d6): 2.67 (s, 1H), 2.07 (s, 1H), 1.44 (s, 1H), 1.28 (d, 2H), 1.26 (s, 2H), 1.23 (d, 2H), 1.08 (d, 1H), 1.04 (d, 1H), 0.80 (s, 6H)

### 15. Preparation of memantine hemi-mucate - not according to the invention

After memantine free base (2.0 g) was added to the reaction unit, dimethyl sulfoxide (20 mL) and acetone (60 mL) were added and dissolved. After adding mucic acid (1.17 g), the temperature was raised to 60°C, and it was stirred for 1 hour, cooled to room temperature, and stirred for 3 hours. The resulting solid was filtered, washed with acetone (10 mL), and then vacuum dried at room temperature overnight to obtain 2.97 g (94%) of the title compound.

1H NMR (400 MHz, DMSO-d6): 3.72 (s, 1H), 3.48 (s, 1H), 3.33 (br, 2H), 2.10 (s, 1H), 1.53 (s, 2H), 1.35 (d, 2H), 1.31 (d, 2H), 1.25 (s, 2H), 1.10 (d, 1H), 1.06 (d, 1H), 0.82 (s, 6H)

### 16. Preparation of memantine hemi-naphthalene disulfonate - not according to the invention

Memantine free base (2.0 g) was added to the reaction unit, and then acetone (20 mL) was added to dissolve it. After adding 1,5-naphthalenedisulfonic acid (4.4 g), the mixture was stirred at room temperature for 3 hours. After adding water (20 mL) to the reaction mixture, the mixture was further stirred for 2 hours. The resulting solid was filtered, washed with a small amount of water, and dried in vacuo at room temperature overnight to obtain 2.50 g (48%) of the title compound.

1H NMR (400 MHz, DMSO-d6): 8.85 (d, 1H), 7.93 (d, 1H), 7.42 (m, 1H), 2.11 (s, 1H), 1.56 (s, 2H), 1.38 (d, 2H), 1.34 (d, 2H), 1.25 (s, 2H), 1.11 (d, 1H), 1.05 (d, 1H), 0.81 (s, 6H)

### 17. Preparation of memantine orotate - not according to the invention

After memantine free base (2.0 g) was added to the reaction unit, acetonitrile (60 mL) was added to dissolve it. After orotic acid (1.94 g) was added, the temperature was raised to 60°C, and the mixture was stirred for 1 hour, cooled to room temperature, and stirred for 3 hours. The resulting solid was filtered, washed with acetonitrile (10 mL), and then vacuum dried at room temperature overnight to obtain 2.78 g (71%) of the title compound.

1H NMR (400 MHz, DMSO-d6): 11.10 (s, 1H), 10.15 (s, 1H), 8.00 (s, 2H), 5.81 (s, 1H), 2.13 (s, 1H), 1.60 (s, 2H), 1.42 (d, 2H), 1.38 (d, 2H), 1.27 (s, 2H), 1.12 (d, 1H), 1,07 (d, 1H), 0.82 (s, 6H)

### 18. Preparation of memantine hemi-oxalate - not according to the invention

Memantine free base (2.0 g) was added to the reaction unit, and then acetone (60 mL) was added to dissolve it. After adding oxalic acid (0.50 g), the temperature was raised to 60°C, and the mixture was stirred for 1 hour, cooled to room temperature, and stirred for 3 hours. The resulting solid was filtered, washed with acetone (10 mL), and then vacuum dried at room temperature overnight to obtain 2.40 g (96%) of the title compound.

1H NMR (400 MHz, DMSO-d6): 2.07 (s, 1H), 1.46 (s, 1H), 1.30 (d, 2H), 1.28 (s, 2H), 1.24 (d, 2H), 1.08 (d, 1H), 1.04 (d, 1H), 0.80 (s, 6H)

### 19. Preparation of memantine hemi-pimelate - not according to the invention

Memantine free base (2.0 g) was added to the reaction unit, and then acetone (60 mL) was added to dissolve it. After adding pimelic acid (0.89 g), the temperature was raised to 60°C, and the mixture was stirred for 1 hour, cooled to room temperature, and stirred for 3 hours. The resulting solid was filtered, washed with acetone (10 mL), and then vacuum dried at room temperature overnight to obtain 2.85 g (99%) of the title compound.

1H NMR (400 MHz, DMSO-d6): 2.07 (m, 2H), 2.04 (s, 1H), 1.44 (m, 2H), 1.38 (s, 2H), 1.19 (s, 2H), 1.17 (d, 2H), 1.05 (d, 1H), 1.02 (d, 1H), 0.79 (s, 6H)

### 20. Preparation of memantine hemi-sebacate - not according to the invention

Memantine free base (2.0 g) was added to the reaction unit, and then acetone (60 mL) was added to dissolve it. After adding sebacic acid (1.13 g), the temperature was raised to 60°C, and the mixture was stirred for 1 hour, cooled to room temperature, and stirred for 3 hours. The resulting solid was filtered, washed with acetone (10 mL), and then vacuum dried at room temperature overnight to obtain 3.06 g (98%) of the title compound.

1H NMR (400 MHz, DMSO-d6): 2.07 (m, 2H), 20.4 (s, 1H), 1.44 (m, 2H), 1.38 (s, 2H), 1.21 (s, 2H), 1.16 (d, 2H), 1.05 (d, 1H), 1.02 (d, 1H), 0.78 (s, 6H)

### 21. Preparation of memantine hemi-suberate - not according to the invention

Memantine free base (2.0 g) was added to the reaction unit, and then acetone (60 mL) was added to dissolve it. After adding suberic acid (0.97 g), the temperature was raised to 60°C, and the mixture was stirred for 1 hour, cooled to room temperature, and stirred for 3 hours. The resulting solid was filtered, washed with acetone (10 mL), and then vacuum dried at room temperature overnight to obtain 2.92 g (98%) of the title compound.

1H NMR (400 MHz, DMSO-d6): 2.09 (m, 2H), 2.03 (s, 1H), 1.43 (m, 2H), 1.35 (s, 2H), 1.21 (s, 2H), 1.18 (d, 2H), 1.14 (d, 2H), 1.04 (d, 1H), 1.02 (d, 1H), 0.78 (s, 6H)

### 22. Preparation of memantine hemi-succinate - not according to the invention

Memantine free base (2.0 g) was added to the reaction unit, and then ethyl acetate (60 mL) was added to dissolve it. After succinic acid (0.66 g) was added, the temperature was raised to 60°C, and the mixture was stirred for 1 hour, cooled to room temperature, and stirred for 3 hours. The resulting solid was filtered, washed with ethyl acetate (10 mL), and dried in vacuo at room temperature overnight to obtain 2.60 g (98%) of the title compound.

1H NMR (400 MHz, DMSO-d6): 2.21 (s, 2H), 2.07 (s, 1H), 1.44 (s, 1H), 1.28 (d, 2H), 1.26 (s, 2H), 1.22 (d, 2H), 1.08 (d, 1H), 1.04 (d, 1H), 0.80 (s, 6H)

### 23. Preparation of memantine hemi-L-tartrate - not according to the invention

Memantine free base (2.0 g) was added to the reaction unit, and then acetone (60 mL) was added to dissolve it. After adding L-tartaric acid (0.84 g), the temperature was raised to 60°C, and it was stirred for 1 hour, cooled to room temperature, and stirred for 3 hours. The resulting solid was filtered, washed with acetone (10 mL), and then vacuum dried at room temperature overnight to obtain 2.79 g (98%) of the title compound.

1H NMR (400 MHz, DMSO-d6): 3.78 (s, 1H), 2.10 (s, 1H), 1.58 (s, 2H), 1.41 (d, 2H), 1.36 (d, 2H), 1.25 (s, 2H), 1.10 (d, 1H), 1.07 (d, 1H), 0.81 (s, 6H)

### 24. Preparation of memantine terephthalate - not according to the invention

Memantine free base (2.0 g) was added to the reaction unit, and then dimethyl sulfoxide (20 mL) was added to dissolve it. After terephthalic acid (2.0 g) was added, the mixture was stirred at room temperature for 3 hours. After adding water (20 mL) to the reaction mixture, the mixture was further stirred for 2 hours. The resulting solid was filtered, washed with a small amount of water, and dried in vacuo at room temperature overnight to obtain 3.64 g (94%) of the title compound.

1H NMR (400 MHz, DMSO-d6): 7.90 (s, 4H), 2.11 (s, 1H), 1.64 (s, 2H), 1.48 (d, 2H), 1.42 (d, 2H), 1.26 (s, 2H), 1.11 (d, 1H), 1.06 (d, 1H), 0.81 (s, 6H)

### 25. Preparation of memantine L-aspartate - not according to the invention

After memantine free base (10.0 g) was added to the reaction unit, methanol (100 mL) was added. After adding L-aspartic acid (7.4 g), the mixture was stirred at 60°C for 1 hour. The mixture was cooled to room temperature and then stirred overnight. The resulting solid was filtered, washed with methanol, and then vacuum dried at room temperature overnight to obtain 9.0 g (52%) of the title compound.

1H NMR (600 MHz, DMSO-d6): 7.96 (br s, 4H), 3.32 (t, 1H), 3.15 (d, 1H), 2.40 (ddd, 1H), 2.15 (ddd, 1H), 2.10 (s, 1H), 1.54 (s, 2H), 1.37 (dd, 2H), 1.31 (dd, 2H), 1.22-1.26 (m, 4H), 1.12 (dd, 1H), 1.06 (dd, 1H), 0.82 (s, 6H)

### 26. Preparation of memantine hemi-pamoate - not according to the invention

Memantine hydrochloride (5.0 g) was added to the reaction unit, and then ethanol (50 mL) was added. Disodium pamoate (9.0 g) was dissolved in ethanol (50 mL) and added to the reaction unit. After stirring at room temperature for 1.5 hours, the mixture was cooled to 5°C and stirred overnight. The resulting solid was filtered, washed with ethanol, and then vacuum dried at room temperature overnight to obtain 1.7 g (14%) of the title compound.

1H NMR (600 MHz, DMSO-d6): 8.19 (d, 1H), 8.15 (s, 1H), 8.02 (s, 3H), 7.63 (d, 1H), 7.10 (dt, 1H), 6.99 (dt, 1H), 4.65 (s, 1H), 2.15 (s, 1H), 1.64 (s, 2H), 1.46 (dd, 2H), 1.40 (dd, 2H), 1.29 (s, 4H), 1.15 (dd, 1H), 1.09 (dd, 1H), 0.84 (s, 6H)

### 27. Preparation of memantine cypionate - not according to the invention

Memantine free base (2.0 g) was added to the reaction unit, and then acetonitrile (15 mL) was added. After adding cypionic acid (1.8 mL), the mixture was stirred at room temperature for 3 hours. The resulting solid was filtered, washed with acetonitrile, and dried under vacuum at room temperature overnight to obtain 3.5 g (96%) of the title compound.

1H NMR (400 MHz, DMSO-d6): 2.08 (m, 3H), 1.67 (m, 3H), 1.55 (m, 3H), 1.45 (s, 3H), 1.40 (s, 2H), 1.22 (m, 6H), 1.06 (m, 4H), 0.79 (s, 6H)

### 28. Preparation of memantine D-glucuronate - not according to the invention

Memantine free base (2.0 g) was added to the reaction unit, and then ethanol (60 mL) was added. After glucuronic acid (2.2 g) was added, the mixture was stirred at 60°C for 1 hour. After stirring at room temperature for 3 hours, the resulting solid was filtered, washed with ethanol, and vacuum dried overnight at room temperature to obtain 3.9 g (91%) of the title compound.

1H NMR (600 MHz, DMSO-d6): 6.04 (s, 1H), 5.30 (bs, 1H), 4.37 (bs, 1H), 3.92 (s, 1H), 3.76 (s, 1H), 3.44 (s, 1H), 2.93 (d, 1H), 2.79 (d, 1H), 2.14 (s, 1H), 1.73 (d, 1H), 1.63 (d, 1H), 1.56 (d, 1H), 1.51 (d, 1H), 1.46 (d, 1H), 1.41 (d, 1H), 1.28 (s, 2H), 1.14 (q, 2H), 0.84 (s, 3H)

### 29. Preparation of memantine hippurate - not according to the invention

After memantine free base (2.0 g) was added to the reaction unit, acetonitrile (80 mL) was added. After adding hippuric acid (2.0 g), the mixture was stirred at 60°C for 1 hour. After stirring at room temperature for 3 hours, the resulting solid was filtered, washed with acetonitrile and vacuum dried overnight at room temperature to obtain 3.9 g (98%) of the title compound.

1H NMR (400 MHz, DMSO-d6): 8.04(bs, 1H), 7.50 (m, 1H), 7.44 (m, 2H), 3.57(d, 2H), 2.09 (s, 1H), 1.54 (s, 2H), 1.30 (d, 2H), 1.28 (s, 2H), 1.24 (d, 2H), 1.10 (d, 1H), 1.04 (d, 1H), 0.81 (s, 6H)

### 30. Preparation of memantine D-gluconate - not according to the invention

After memantine free base (10.0 g) was added to the reaction unit, acetone (100 mL) was added. After gluconic acid (3.4 mL) was added, the mixture was stirred at 60°C for 1 hour. After stirring at room temperature for 3 hours, the resulting solid was filtered, washed with acetone, and vacuum dried overnight at room temperature to obtain 2.8 g (13%) of the title compound.

1H NMR (400 MHz, DMSO-d6): 3.74 (d, 1H), 3.58 (d, 1H), 3.57 (d, 1H), 3.46 (s, 1H), 3.40 (d, 1H), 3.32 (m, 2H), 2.12 (s, 1H), 1.57 (s, 1H), 1.36 (d, 2H), 1.27 (s, 2H), 1.24 (d, 2H), 1.13 (d, 1H), 1.06 (d, 1H), 0.83 (s, 6H)

### 31. Preparation of memantine laurate - not according to the invention

After memantine free base (1 g) was added to the reaction unit, normal hexane (15 mL) was added. After adding lauric acid (1.12 g), the mixture was stirred at room temperature for 1 hour. The reaction product was concentrated under reduced pressure and then vacuum dried at room temperature overnight to obtain 2.12 g (100%) of the title compound.

1H NMR (600 MHz, CD3OD): 4.97 (s, 2H), 2.24 (m, 1H), 2.15 (t, 2H), 1.71 (s, 2H), 1.59 (m, 2H), 1.52 (d, 2H), 1.47 (d, 2H), 1.41 (s, 4H), 1.24-1.32 (m, 17H), 1.19 (d, 1H), 0.89-0.92 (m, 9H)

### 32. Preparation of memantine palmitate - not according to the invention

After memantine free base (1 g) was added to the reaction unit, normal hexane (15 mL) was added. After adding palmitic acid (1.43 g), the mixture was stirred at room temperature for 1 hour. The reaction product was concentrated under reduced pressure and then vacuum dried at room temperature overnight to obtain 2.43 g (100%) of the title compound.

1H NMR (600 MHz, CD3OD): 4.96 (s, 2H), 2.24 (m, 1H), 2.15 (t, 2H), 1.71 (s, 2H), 1.59 (m, 2H), 1.52 (d, 2H), 1.46 (d, 2H), 1.41 (s, 4H), 1.24-1.32 (m, 25H), 1.19 (d, 1H), 0.89-0.92 (m, 9H)

### 33. Preparation of memantine decanoate - not according to the invention

After memantine free base (1 g) was added to the reaction unit, normal hexane (15 mL) was added. After adding decanoic acid (0.96 g), the mixture was stirred at room temperature for 1 hour. The reaction product was concentrated under reduced pressure and then vacuum dried at room temperature overnight to obtain 1.96 g (100%) of the title compound.

1H NMR (600 MHz, CD3OD): 5.01 (s, 2H), 2.24 (m, 1H), 2.15 (t, 2H), 1.71 (s, 2H), 1.60 (m, 2H), 1.53 (d, 2H), 1.47 (d, 2H), 1.41 (s, 4H), 1.23-1.32 (m, 13H), 1.19 (d, 1H), 0.89-0.92 (m, 9H)

### 34. Preparation of memantine myristate - not according to the invention

After memantine free base (1 g) was added to the reaction unit, normal hexane (15 mL) was added. After adding myristic acid (1.27 g), the mixture was stirred at room temperature for 1 hour. The reaction product was concentrated under reduced pressure and then vacuum dried at room temperature overnight to obtain 2.27 g (100%) of the title compound.

1H NMR (600 MHz, CD3OD): 4.95 (s, 2H), 2.24 (m, 1H), 2.15 (t, 2H), 1.71 (s, 2H), 1.59 (m, 2H), 1.52 (d, 2H), 1.46 (d, 2H), 1.41 (s, 4H), 1.24-1.32 (m, 21H), 1.19 (d, 1H), 0.89-0.92 (m, 9H)

### <Experimental Example 1> Solubility evaluation for the solubilizer propylene glycol monocaprylate

The solubility of the various memantine salts prepared in 1 to 34 of the above Preparation Example 1 in solubilizers was evaluated, in order to select memantine salts that can be used for percutaneous absorption preparation.

In order to evaluate the solubility of memantine salt in the solubilizer, solubility evaluation was performed for propylene glycol monocaprylate. For solubility evaluation, to 1 mL of propylene glycol monocaprylate, 50 mg of memantine free base was added in the form of the memantine salts prepared in 1 to 34 of Preparation Example 1. The resulting mixture was treated in a shaking constant temperature bath at 25°C for 24 hours. Afterward, the supernatant was separated and centrifuged at 25°C and 13000 rpm for 25 minutes, and the supernatant was passed through a 0.45um filter. The filtrate was diluted in ethanol at an appropriate ratio. Then, into a 50 mL volumetric flask, 5 mL of the diluted solution or 5 mL of standard solution (memantine hydrochloride 1g/L), 4 mL of 0.015 M FMOC (fluorenylmethoxycarbonyl chloride) solution, 4 mL of 0.05 M pH8.5 borate buffer were added and mixed. Then, diluent (0.05 M pH8.5 borate buffer: acetonitrile = 1:1) was added to adjust the total volume to 50 mL. The resulting mixture was left at room temperature for 20 minutes.

Memantine solubility of this solution was calculated by liquid chromatography, and the range is shown in Table 1.

### <Liquid chromatography conditions>

Column: Kromasil C18, 4.6 X 150 mm, 5 um
Mobile Phase: pH 3.0 Potassium Phosphate Buffer: Acetonitrile = 20: 80
Column temperature: 30°C
Flow rate: 2.0 mL/min
Injection size: 20 uL
UV wavelength: 265 nm

As can be seen in Table 1 below, according to the solubility evaluation result of the memantine salts prepared in Preparation Example 1 (1 to 34) for the solubilizer propylene glycol monocaprylate, enanthate, lactate, cypionate, caprylate, laurate, levulinate, palmitate, valerate, decanoate, and myristate salts show high solubility of 30mg/mL or more, indicating that they can be made into percutaneous absorption preparation.

**[Table 1]**

| Solubility screening results for propylene glycol monocaprylate | |
|---|---|
| Memantine salt | Propylene glycol monocaprylate solubility (mg/mL) |
| hydrochloride | <5 |
| free base | >50 |
| enanthate | >50 |
| lactate | >50 |
| cypionate | <50 |
| caprylate | <50 |
| laurate | <45 |
| levulinate | <45 |
| palmitate | <45 |
| valerate | <45 |
| decanoate | <40 |
| myristate | <40 |
| xinafoate | <20 |
| (+)-camphorsulfonate | <10 |
| hemi-adipate | <5 |
| hemi-edisylate | <5 |
| hemi-fumarate | <5 |
| hemi-malate | <5 |
| hemi-maleate | <5 |
| hemi-malonate | <5 |
| hemi-mucate | <5 |
| hemi-glutamate | <5 |
| orotate | <5 |
| hemi-oxalate | <5 |
| hemi-pimelate | <5 |
| Hemi-naphthalene disulfonate | <5 |
| hemi-sebacate | <5 |
| hemi-suberate | <5 |
| hemi-succinate | <5 |
| hemi-L-tartrate | <5 |
| terephthalate | <5 |
| L-aspartate | <5 |
| hemi-pamoate | <5 |
| hippurate | <5 |
| D-glucuronate | <5 |
| D-gluconate | <5 |

### <Experimental Example 2> Screening evaluation of oil phase skin permeability for solubilizer

Based on the solubility test results of various memantine salts evaluated in Experimental Example 1, memantine salts having a solubility of 30 mg/mL or more for solubilizer propylene glycol monocaprylate were selected, and in-vitro permeability evaluation was performed. A pH 7.4 phosphate buffer containing 10% ethanol and 0.02% sodium azide as an aqueous solution was added to the receptor chamber of the Franz diffusion cell, and with the temperature maintained at 32 ± 0.5°C, it was rotated at 600 rpm and stirred. Human cadaver skin was fixed between the donor chamber and the receptor chamber in a Franz diffusion cell. 200ul of memantine salt-solubilizer solution at a concentration of 50mg/mL was evenly applied on the skin, and after permeation for 7 days, the amount of memantine permeation was measured by liquid chromatography, and the results are shown in Table 2 below.

As can be seen in Table 2 below, according to the evaluation results of oil phase skin permeability of each memantine salt dissolved in propylene glycol monocaprylate, the levulinate, lactate, and enanthate showed a skin permeation of about 3000 ug/cm2 or more for 7 days and a skin permeation rate of 18 ug/cm2/hr or more for 7 days, which was 1.4 to 1.7 times higher than that of the free base, whereas the caprylate, laurate, palmitate, cypionate, decanoate, and myristate salts showed lower skin permeability compared to the free base. The levulinate, lactate, and enanthate showed sufficient skin permeability for percutaneous absorption preparation, indicating that they can be used to design percutaneous absorption preparation.

**[Table 2]**

| Oil phase skin permeability screening result | | |
|---|---|---|
| Salt type | Skin permeability for 7 days (ug/cm2) | Skin penetration rate (ug/cm2/hr) |
| **Levulinate** | **3661** | **22** |
| **Lactate** | **3655** | **22** |
| **Enanthate** | **2969** | **18** |
| **Free base** | **2102** | **13** |
| Caprylate | 2094 | 12 |
| Cypionate | 2060 | 12 |
| Decanoate | 1257 | 7 |
| Myristate | 1083 | 6 |
| Laurate | 1019 | 6 |
| Palmitate | 873 | 5 |

### <Experimental Example 3> Evaluation of solubility of memantine according to the type of solubilizer

In order to design a percutaneous absorption preparation of memantine enanthate selected in Experimental Example 2, solubility with various solubilizers was evaluated. Furthermore, the levulinate, disclosed in prior art literature, was chosen for comparison, though Experimental Example 2 results showed that the design of a percutaneous absorption preparation was possible. For solubility evaluation, an excess of memantine enanthate and memantine levulinate was added to 1 mL of each solubilizer and treated in a shaking water bath at 25°C for 24 hours. Then, the supernatant was separated, and the supernatant was centrifuged at 25°C and 13000 rpm for 25 minutes. After centrifugation, the supernatant was passed through a 0.45um filter. The filtrate was diluted in ethanol at an appropriate ratio. Then, into a 50 mL volumetric flask, 5 mL of the diluted solution or 5 mL of standard solution (memantine hydrochloride 1g/L), 4 mL of 0.015 M FMOC solution, 4 mL of 0.05 M pH8.5 borate buffer were added, and mixed. Then, a diluent (0.05 M pH8.5 borate buffer: acetonitrile = 1:1) was added to adjust the total volume to 50 mL. The resulting mixture was left at room temperature for 20 minutes.

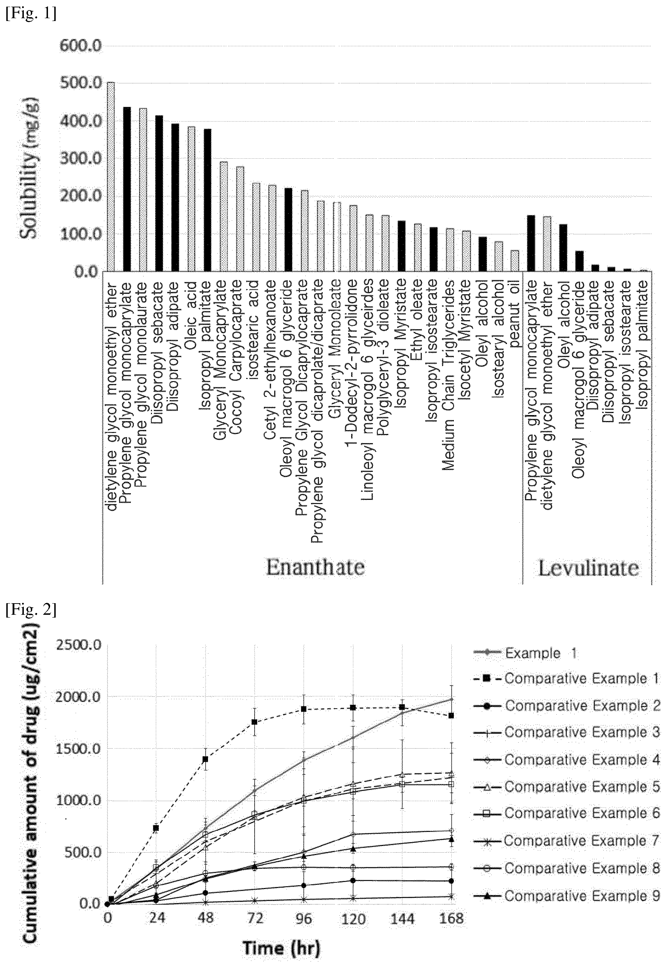

Liquid chromatography was performed to measure the solubility, and the result is shown in Figure 1.

As seen in Figure 1, memantine enanthate showed higher overall solubility with the solubilizers than memantine levulinate. In general, if a drug has low solubility with the solubilizer in percutaneous absorption preparations, it may cause insufficient release of the drug due to crystal precipitation, and using a large amount of solubilizer to sufficiently dissolve the drug may lead to undesirable properties of the resulting formulation. Therefore, it would be advantageous to design a percutaneous absorption preparation comprising memantine enanthate, which has higher solubility with various solubilizers.

Additionally, in order to compare the percutaneous permeability of memantine enanthate, free base, and other salts, 8 solubilizers having various solubility were selected, and percutaneous absorption preparations comprising memantine and its salts were prepared, as shown in the following Examples and Comparative Examples.

### <Example 1> Preparation of memantine percutaneous absorption preparation according to the present invention

A percutaneous absorption preparation comprising memantine enanthate of the present invention was prepared as Example 1 as follows. With the composition shown in Table 3 below, 17.6 g of a styrene-isoprene-styrene block copolymer, 7 g of octyldodecyl myristate, and 6.5 g of propylene glycol monocaprylate were dissolved in 32 g of ethyl acetate, and 3.45 g of memantine enanthate was added thereto and dissolved homogeneously. The resulting solution was applied to a silicone-coated PET film so that the drug content per unit area was 3.0 mg/cm2 as memantine, dried in an oven at 80°C for 30 minutes, and then laminated with a backing film to prepare a percutaneous absorption preparation.

**[Table 3]**

| | **Ingredient (g)** | **Example 1** |
|---|---|---|
| **Active ingredient** | Memantine enanthate | 3.45 |
| **Adhesive** | Styrene-isoprene-styrene block copolymer | 17.6 |
| **Tackifier** | Terpene resin | 6 |
| **Solubilizer** | Propylene glycol monocaprylate | 6.5 |
| **Plasticizer** | Octyldodecyl Myristate | 7 |
| | Total | 40.55 |

### <Examples 2 to 8> Preparation of memantine percutaneous absorption preparation for each solubilizer

As described in Experimental Example 3, 8 types of solubilizers having various memantine solubility were selected based on the solubility results. For Examples 2 through 8, memantine-containing percutaneous absorption preparations were prepared in the same way as Example 1 but with different solubilizers according to the compositions shown in Table 4 below.

**[Table 4]**

| | **Ingredient (g)** | **Example 2** | **Example 3** | **Example 4** | **Example 5** | **Example 6** | **Example 7** | **Example 8** |
|---|---|---|---|---|---|---|---|---|
| **Active ingredient** | Memantine enanthate | 3.45 | 3.45 | 3.45 | 3.45 | 3.45 | 3.45 | 3.45 |
| **Adhesive** | Styrene-isoprene-styrene block copolymer | 17.6 | 17.6 | 17.6 | 17.6 | 17.6 | 17.6 | 17.6 |
| **Tackifier** | Terpene resin | 6 | 6 | 6 | 6 | 6 | 16 | 6 |
| **Solubilizer** | Diisopropyl sebacate | 6.5 | | | | | | |
| | diisopropyl adipate | | 6.5 | | | | | |
| | Isopropyl palmitate | | | 6.5 | | | | |
| | Isopropyl myristate | | | | 6.5 | | | |
| | Oleoyl macrogol-6-glyceride | | | | | 6.5 | | |
| | Isopropyl isostearate | | | | | | 6.5 | |
| | Oleyl alcohol | | | | | | | 6.5 |
| **Plasticizer** | Octyldodecyl myristate | 7 | 7 | 7 | 7 | 7 | 7 | 7 |
| | Total | 40.55 | 40.55 | 40.55 | 40.55 | 40.55 | 40.55 | 40.55 |

### <Examples 9 to 11> Preparation of memantine percutaneous absorption preparations of the present invention with reduced solubilizer content and with different adhesive

For Examples 9 through 11, percutaneous absorption preparations comprising memantine were prepared in the same manner as Example 1 but with the composition described in Table 5 below. Example 9 was prepared by changing the amount of solubilizer from Example 1. Example 10 was prepared by changing the amount of solubilizer from Example 3. Example 11 was prepared by changing the adhesive from Example 3.

**[Table 5]**

| | **Ingredient (g)** | **Example 9** | **Example 10** | **Example 11** |
|---|---|---|---|---|
| **Active ingredient** | Memantine enanthate | 3.45 | 3.45 | 3.45 |
| **Adhesive** | Styrene-isoprene-styrene block copolymer | 17.6 | 17.6 | |
| | Durotak 87-9301 | | | 30.6 |
| **Tackifier** | Terpene resin | 6 | 6 | |
| **Solubilizer** | Propylene glycol monocaprylate | 3.5 | | |
| | Diisopropyl adipate | | 3.5 | 6.5 |
| **Plasticizer** | Octyldodecyl myristate | 7 | 7 | |
| | Total | 37.55 | 37.55 | 40.55 |

### <Example 12> Preparation of memantine percutaneous absorption preparation of present invention with changes in the solubilizer and the adhesive

For Example 12, percutaneous absorption preparation was prepared according to the components and contents shown in Table 6 below, which is the example described in U.S. Patent Application Publication No. 2019-0183810, with memantine enanthate added instead of memantine levulinate.

After dissolving 5.61 g of memantine enanthate and 3 g of propylene glycol in 26.9 g of toluene and 0.6 g of isopropyl alcohol, 1.75 g of fumed silica was added and dispersed, and then 24.8 g of polyisobutylene (14.9 g as a solid) was added, and the resulting mixture was stirred to prepare the drug mixture. The resulting solution was applied to a silicone-coated PET film, dried in an oven at 60°C for 30 minutes, and then laminated with a backing film to prepare a drug layer. After preparing an adhesive layer mixture in the same manner, it was applied to a silicone-coated PET film and dried, and then a drug layer from which the PET film was removed was laminated on the adhesive layer to prepare Example 12.

**[Table 6]**

| **Ingredient (g)** | **Example 12** | |
|---|---|---|
| | Drug layer | Adhesive layer |
| **Memantine enanthate** | 5.61 | 4.01 |
| **Polyisobutylene polymer** | 14.90 | 17.78 |
| **Propylene glycol** | 3 | 2.15 |
| **Fumed silica (Aerosil 200P)** | 1.75 | 1.25 |
| **Total** | 25.3 | 25.2 |

### <Comparative Examples 1 to 4> Preparation of percutaneous absorption preparations with other memantine salt and free base

To compare the characteristics of memantine enanthate with other salts, various percutaneous absorption preparations were prepared according to the composition shown in Table 7. Comparative Examples 1 through 4 were prepared in the same manner as in Example 1 but with memantine free base, memantine hydrochloride used in commercially available products, and memantine levulinate and memantine lactate, which were evaluated in Experimental Example 2 to be suitable for designing percutaneous absorption preparations. In addition, a percutaneous absorption preparation having the same composition as in Comparative Example 1 with only a different amount of active ingredient (2 mg/cm2) was prepared as Comparative Example 1-1.

**[Table 7]**

| | **Ingredient (g)** | **Comparative Example 1** | **Comparative Example 1-1** | **Comparative Example 2** | **Comparative Example 3** | **Comparative Example 4** |
|---|---|---|---|---|---|---|
| **Active ingredient** | Memantine free base | 2 | 1.33 | | | |
| | Memantine hydrochloride | | | 2.4 | | |
| | Memantine levulinate | | | | 3.3 | |
| | Memantine lactate | | | | | 3 |
| **Adhesive** | Styrene-isoprene-styrene block copolymer | 17.6 | 17.6 | 17.6 | 17.6 | 17.6 |
| **Tackifier** | Terpene resin | 6 | 6 | 6 | 6 | 6 |
| **Solubilizer** | Propylene glycol monocaprylate | 6.5 | 6.5 | 6.5 | 6.5 | 6.5 |
| **Plasticizer** | Octyldodecyl myristate | 7 | 7 | 7 | 7 | 7 |
| | Total | 39.1 | 38.43 | 39.5 | 40.4 | 40.1 |

### <Comparative Example 5> Preparation of percutaneous absorption preparation comprising memantine free base and levulinate

For Comparative Example 5, a patch was prepared in which levulinic acid was added to memantine free base to form a salt, according to the components and contents shown in Table 8 below. It is an example described in US Patent Application Publication No. 2019-0183810. Specifically, Comparative Example 5 was prepared as follows. 3.25 g of memantine free base, 2.1 g of levulinic acid and 3 g of propylene glycol were dissolved in 26.9 g of toluene and 0.6 g of isopropyl alcohol, and 1.75 g of fumed silica was added and dispersed, followed by adding of 24.8 g of polyisobutylene (14.9 g as a solid). Then, the resulting mixture was stirred. The resulting solution was applied to a silicone-coated PET film, dried in an oven at 60°C for 30 minutes, and then laminated with a backing film to prepare a drug layer. An adhesive layer mixture was prepared in the same way and was applied to a silicone-coated PET film and dried, and then a drug layer from which the PET film was removed was laminated on the adhesive layer to prepare Comparative Example 5.

**[Table 8]**

| **Ingredient (g)** | **Comparative Example 5** | |
|---|---|---|
| | **Drug layer** | **Adhesive layer** |
| **Memantine free base** | 3.25 | 2.325 |
| **Polyisobutylene polymer** | 14.90 | 17.78 |
| **Propylene glycol** | 3 | 2.15 |
| **Levulinic acid** | 2.1 | 1.5 |
| **Fumed silica (Aerosil 200P)** | 1.75 | 1.25 |
| **Total** | 25 | 25 |

### <Comparative Example 6> Preparation of a double-layer in-situ percutaneous absorption preparation comprising memantine hydrochloride and sodium bicarbonate

For Comparative Example 6, memantine hydrochloride in-situ percutaneous absorption preparation was prepared with the components and contents shown in Table 9 below. It is an example described in Korean Patent Application Publication No. 2019-0032551. Specifically, 3 g of octyldodecanol and 3 g of glycerol are dissolved in 44 g of ethyl acetate and 2.79 g of isopropyl alcohol, 7.5 g of memantine hydrochloride and 2.9 g of sodium bicarbonate are added and stirred, and 4.5 g of Kollidone CL-M is added and dispersed. Then, 23.3 g of Durotak 87-4287 (9.1 g as a solid) was added and the resulting mixture was stirred. The resulting solution was applied to a silicone-coated PET film, dried in an oven at 60°C for 30 minutes, and then laminated with a backing film to prepare a drug layer. An adhesive layer mixture was prepared in the same way and was applied to a silicone-coated PET film and dried, and then a drug layer from which the PET film was removed was laminated on the adhesive layer to prepare Comparative Example 6.

**[Table 9]**

| **Ingredient (g)** | **Comparative Example 6** | |
|---|---|---|
| | **Drug layer** | **Adhesive layer** |
| **Memantine hydrochloride** | 7.5 | |
| **Sodium bicarbonate** | 2.90 | |
| **Octyldodecanol** | 3 | 2 |
| **Glycerol** | 3 | |
| **Kollidon CL-M** | 4.5 | 4 |
| **Durotak 87-4287** | 9.1 | |
| **Polyisobutylene polymer** | | 14.0 |
| **Total** | 30.0 | 20.0 |

### <Comparative Example 7> Preparation of percutaneous absorption preparation comprising memantine free base and release-controlling agent

For Comparative Example 7, a patch was prepared comprising memantine free base and a release controlling agent, with the components and contents shown in Table 10 below. This is an example described in Korean Patent No. 10-1964295. Specifically, Comparative Example 7 was prepared as follows. 0.02 g of dibutylhydroxytoluene, 1 g of PVP 90F, and 1 g of stearate were dissolved in 2 g of ethanol, and then 2 g of memantine free base, 43.78 g of acrylate polymer (87-9301) (amount of mixture corresponding to 15.98 g as a solid) was added to prepare an adhesive acrylate drug mixture. The resulting solution was applied to a silicone-coated PET film, dried at room temperature for 10 minutes, dried in an oven at 70°C for 20 minutes, and then laminated with a backing film to prepare a drug layer. A release control layer mixture was prepared in the same way, and it was applied to a silicone-coated PET film and dried, and then a drug layer from which the PET film was removed was laminated on the release control layer to prepare Comparative Example 7.

**[Table 10]**

| **Ingredient (g)** | **Comparative Example 7** | |
|---|---|---|
| | **Drug layer** | **Release control layer** |
| **Memantine free base** | 2 | |
| **Dibutylhydroxytoluene** | 0.02 | 0.02 |
| **Stearic acid** | 1 | |
| **PVP 90F** | 1 | |
| **Durotak 87-9301** | 15.98 | |
| **Bio-PSA 4201** | | 19.58 |
| **Silicone oil** | | 0.4 |
| **Total** | 20 | 20 |

### <Comparative Example 8> Preparation of single-layer in-situ percutaneous absorption preparation comprising memantine hydrochloride and sodium ethanolate

For Comparative Example 8, memantine hydrochloride in-situ percutaneous absorption preparation was prepared with the components and contents shown in Table 11 below, which is an example described in U.S. Patent No. 8,882,729. Specifically, 2 g of memantine hydrochloride was suspended in 0.66 g of ethyl acetate, and 41 g of Durotak 87-2516 (16.6 g as a solid) was added. After stirring, 1.4 g of sodium ethanolate was added and stirred to prepare a drug mixture. The resulting solution was applied to a silicone-coated PET film, dried in an oven at 80°C for 30 minutes, and then laminated with a backing film to prepare Comparative Example 8.

**[Table 11]**

| | **Comparative Example 8** |
|---|---|
| **Memantine hydrochloride** | 2 |
| **Sodium ethanolate** | 1.4 |
| **Duro-Tak 87-2516** | 16.6 |
| **Total** | 20 |

### <Comparative Example 9> Preparation of percutaneous absorption preparation comprising memantine caprylate and acrylic pressure-sensitive adhesive

For Comparative Example 9, a percutaneous absorption preparation comprising memantine caprylate was prepared with the components and contents shown in Table 12 below, as described in an example in International Patent Application Publication WO 2014-174564. Specifically, 0.46 g of NaOH was dissolved in 7.71 mL of methanol, and 2.5 g of memantine hydrochloride is added and mixed. Then, 2.00 g of caprylic acid is added and mixed, and 17.5 g of Toluene and 51.39 g of Durotak 87-4287 (20.04 g as a solid) were added. After stirring, the mixture was applied to a silicone-coated PET film, dried in an oven at 80°C for 30 minutes, and laminated with a backing film to prepare Comparative Example 9.

**[Table 12]**

| **Ingredient (g)** | **Comparative Example 9** |
|---|---|
| **sodium hydroxide** | 0.46 |
| **Memantine hydrochloride** | 2.5 |
| **Caprylic acid** | 2 |
| **Duro-Tak 87-4287 (39%)** | 20.04 |
| **Total** | 25 |

### <Comparative Examples 10 to 15> Preparation of percutaneous absorption preparations comprising different salts and free base of memantine for solubilizers diisopropyl sebacate and diisopropyl adipate

To compare the characteristics of memantine enanthate with other salts, Comparative Examples 10 through 15 were prepared in the same manner as in Example 1 but according to the compositions shown in Table 13 below. In Comparative Examples 10 through 12, the salt of the active ingredient was changed from Example 2 of the present invention; and in Comparative Examples 13 through 15, the salt of the active ingredient was changed from that of Example 3.

**[Table 13]**

| | **Ingredient (g)** | **Comparative Example 10** | **Comparative Example 11** | **Comparative Example 12** | **Comparative Example 13** | **Comparative Example 14** | **Comparative Example 15** |
|---|---|---|---|---|---|---|---|
| **Active ingredient** | Memantine free base | 2 | | | 2 | | |
| | Memantine hydrochloride | | 2.4 | | | 2.4 | |
| | Memantine levulinate | | | 3.3 | | | 3.3 |
| **Adhesive** | Styrene-isoprene-styrene block copolymer | 17.6 | 17.6 | 17.6 | 17.6 | 17.6 | 17.6 |
| **Tackifier** | Terpene resin | 6 | 6 | 6 | 6 | 6 | 6 |
| **Solubilizer** | Diisopropyl sebacate | 6.5 | 6.5 | 6.5 | | | |
| | Diisopropyl adipate | | | | 6.5 | 6.5 | 6.5 |
| **Plasticizer** | Octyldodecyl myristate | 7 | 7 | 7 | 7 | 7 | 7 |
| | Total | 39.1 | 39.5 | 40.4 | 39.1 | 39.5 | 40.4 |

### <Comparative Examples 16 to 21> Preparation of percutaneous absorption preparations comprising different salts and free base of memantine for solubilizers isopropyl palmitate and isopropyl myristate

To compare the characteristics of memantine enanthate with other salts, Comparative Examples 16 through 21 were prepared in the same manner as in Example 1 but according to the compositions shown in Table 14 below. In Comparative Examples 16 through 18, the salt of the active ingredient was changed from Example 4 of the present invention; and in Comparative Examples 19 through 21, the salt of the active ingredient was changed from that of Example 5.

**[Table 14]**

| | **Ingredient (g)** | **Comparative Example 16** | **Comparative Example 17** | **Comparative Example 18** | **Comparative Example 19** | **Comparative Example 20** | **Comparative Example 21** |
|---|---|---|---|---|---|---|---|
| **Active ingredient** | Memantine free base | 2 | | | 2 | | |
| | Memantine hydrochloride | | 2.4 | | | 2.4 | |
| | Memantine levulinate | | | 3.3 | | | 3.3 |
| **Adhesive** | Styrene-isoprene-styrene block copolymer | 17.6 | 17.6 | 17.6 | 17.6 | 17.6 | 17.6 |
| **Tackifier** | Terpene resin | 6 | 6 | 6 | 6 | 6 | 6 |
| **Solubilizer** | Isopropyl palmitate | 6.5 | 6.5 | 6.5 | | | |
| | Isopropyl myristate | | | | 6.5 | 6.5 | 6.5 |
| **Plasticizer** | Octyldodecyl myristate | 7 | 7 | 7 | 7 | 7 | 7 |
| | Total | 39.1 | 39.5 | 40.4 | 39.1 | 39.5 | 40.4 |

### <Comparative Examples 22 to 27> Preparation of percutaneous absorption preparations comprising memantine free base and different salts for solubilizers oleoyl macrogol-6- glyceride and isopropyl isostearate

To compare the characteristics of memantine enanthate with other salts, Comparative Examples 22 through 27 were prepared in the same manner as in Example 1 but according to the compositions shown in Table 15 below. In Comparative Examples 22 through 24, the salt of the active ingredient was changed from Example 6 of the present invention; and in Comparative Examples 25 through 27, the salt of the active ingredient was changed from that of Example 7.

**[Table 15]**

| | **Ingredient (g)** | **Comparative Example 22** | **Comparative Example 23** | **Comparative Example 24** | **Comparative Example 25** | **Comparative Example 26** | **Comparative Example 27** |
|---|---|---|---|---|---|---|---|
| **Active ingredient** | Memantine free base | 2 | | | 2 | | |
| | Memantine hydrochloride | | 2.4 | | | 2.4 | |
| | Memantine levulinate | | | 3.3 | | | 3.3 |
| **Adhesive** | Styrene-isoprene-styrene block copolymer | 17.6 | 17.6 | 17.6 | 17.6 | 17.6 | 17.6 |
| **Tackifier** | Terpene resin | 6 | 6 | 6 | 6 | 6 | 6 |
| **Solubilizer** | Oleoyl macrogol-6-glyceride | 6.5 | 6.5 | 6.5 | | | |
| | Isopropyl isostearate | | | | 6.5 | 6.5 | 6.5 |
| **Plasticizer** | Octyldodecyl myristate | 7 | 7 | 7 | 7 | 7 | 7 |
| | Total | 39.1 | 39.5 | 40.4 | 39.1 | 39.5 | 40.4 |

### <Comparative Examples 28 to 30> Preparation of percutaneous absorption preparations with memantine free base and other salts with solubilizer oleyl alcohol

To compare the characteristics of memantine enanthate with other salts, Comparative Examples 28 through 30 were prepared in the same manner as in Example 1 but according to the compositions shown in Table 16 below, where the salt of the active ingredient was changed from Example 8 of the present invention.

**[Table 16]**

| | **Ingredient (g)** | **Comparative Example 28** | **Comparative Example 29** | **Comparative Example 30** |
|---|---|---|---|---|
| **Active ingredient** | Memantine free base | 2 | | |
| | Memantine hydrochloride | | 2.4 | |
| | Memantine levulinate | | | 3.3 |
| **Adhesive** | Styrene-isoprene-styrene block copolymer | 17.6 | 17.6 | 17.6 |
| **Tackifier** | Terpene resin | 6 | 6 | 6 |
| **Solubilizer** | Oleyl alcohol | 6.5 | 6.5 | 6.5 |
| **Plasticizer** | Octyldodecyl myristate | 7 | 7 | 7 |
| | Total | 39 1 | 39.5 | 40.4 |

### <Experimental Example 5> Evaluation of skin permeability of memantine percutaneous absorption preparation

In-vitro permeability evaluation of each of the percutaneous absorption preparations prepared in Examples 1 to 10 and Comparative Examples 1 to 30 was performed using a Franz diffusion cell. A pH 7.4 phosphate buffer containing 10% Ethanol and 0.02% sodium azide was added as an aqueous solution to the receptor chamber, and with the temperature maintained at 32±0.5°C, the mixture was stirred at 600 rpm. Human cadaver skin was fixed between the donor chamber and the receptor chamber in a Franz diffusion cell. Each of the percutaneous absorption preparations prepared in Examples 1 to 10 and Comparative Examples 1 to 30 were cut to fit the donor cell size and applied to the skin, and the amount of memantine permeation over time was measured by liquid chromatography. Examples 1 through 10 and Comparative Examples 1 through 30 show the drug release pattern according to ttime of the patch prepared in Figures 2 through 10.

### 1. Comparative evaluation of skin permeability of percutaneous absorption preparations according to the type of memantine salt

As shown in Figure 2, Comparative Example 2 comprising memantine hydrochloride had skin permeability of 199.3 ug/cm2 for 7 days, indicating that memantine had hardly permeated; Comparative Example 3 comprising memantine levulinate and Comparative Example 4 comprising memantine lactate had higher skin permeability than Comparative Example 2, but 7-day skin permeability result was 1217.9 ug/cm2 and 711.1 ug/cm2, respectively, which was less than half of the content; and even in comparison to Comparative Examples 5 through 9 from prior art, Example 1 of the present invention showed higher skin permeability of at least 1.56 times more.

On the other hand, Example 1 comprising memantine enanthate shows a 7-day skin permeability of 1972.6 ug/cm2, which was 1.6 to 9.9 times that of Comparative Examples 2 through 4, indicating that the skin permeability was remarkably high.

Furthermore, although Comparative Example 1 comprising memantine free base exhibited a skin permeability of 1814.8 ug/cm2, it showed rapid skin permeation for a short period of time, causing severe skin irritation. On the other hand, Example 1 comprising memantine enanthate showed a constant skin permeation and release pattern for a long time.

Therefore, it was found that the percutaneous absorption preparation comprising memantine enanthate of the present invention has the advantage of being useful for designing a sustained-release formulation as well as minimizing human skin irritation.

### 2. Evaluation of skin permeability of percutaneous absorption preparations according to solubilizer solubility and memantine salt

As mentioned in the solubility test result of Experimental Example 3, to confirm that the percutaneous absorption preparations comprising memantine enanthate exhibited high skin permeability and a constant release pattern regardless of the solubility of the solubilizer used, solubilizers of various solubility were added to evaluate the skin permeability of the prepared Examples and Comparative Examples.

The solubilizers having a solubility of 50 mg/g or more were selected, which were diisopropyl sebacate, diisopropyl adipate, isopropyl palmitate, isopropyl myristate, isopropyl isostearate, and oleyl alcohol.

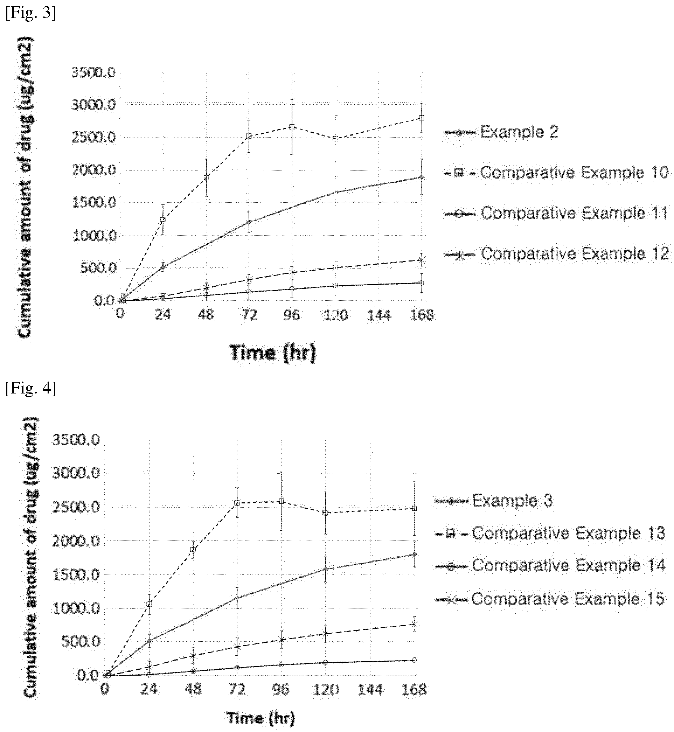

As shown in Figure 3, Example 2, which had diisopropyl sebacate added as a solubilizer, exhibited the same skin permeability results as Example 1, in which propylene glycol monocaprylate was added as a solubilizer, and also maintained higher skin permeability compared to Comparative Example 11 (comprising memantine hydrochloride) and Comparative Example 12 (containing memantine levulinate). In addition, Example 2 showed a constant skin permeation and release pattern for a long time compared to Comparative Example 10 (comprising memantine free base), similar to the result in Figure 2.

As shown in Figure 4, Example 3, which had diisopropyl adipate added as a solubilizer, showed the same level of skin permeability as Example 1, and maintained higher skin permeability than Comparative Example 14 (comprising memantine hydrochloride) and Comparative Example 15 (comprising memantine levulinate). In addition, Example 3 showed a constant skin permeation and release pattern for a long time compared to Comparative Example 13 (comprising memantine free base).

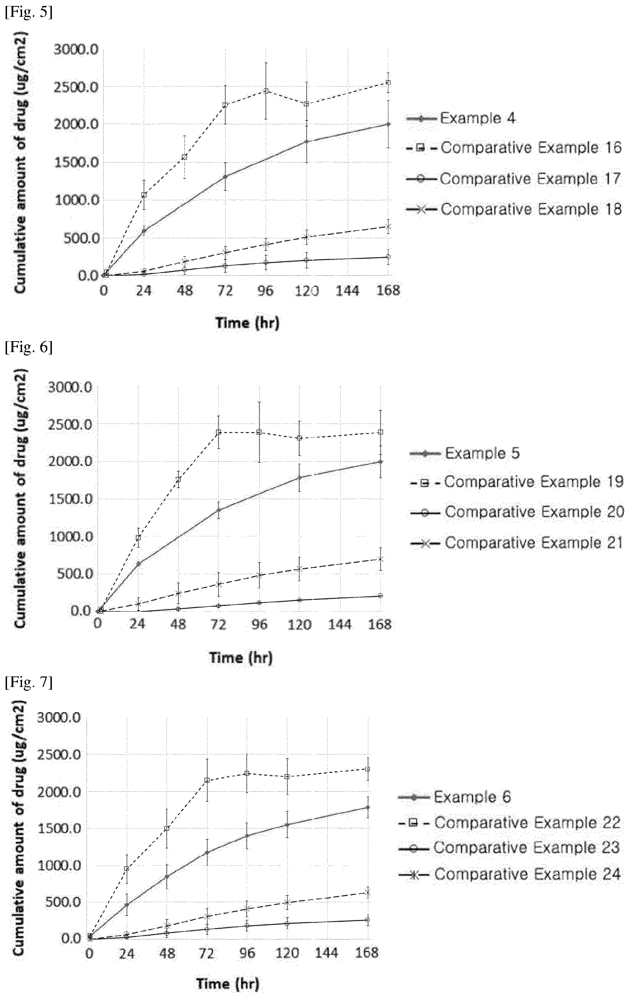

As shown in Figure 5, Example 4, which had isopropyl palmitate added as a solubilizer, showed the same level of skin permeability as Example 1 and maintained higher skin permeability than Comparative Example 17 (comprising memantine hydrochloride) and Comparative Example 18 (comprising memantine levulinate). In addition, Example 4 showed a constant skin permeation and release pattern for a long time compared to Comparative Example 16 (comprising memantine free base).

As shown in Figure 6, Example 5, which had isopropyl myristate added as a solubilizer, showed the same level of skin permeability as Example 1 and higher skin permeability than Comparative Example 20 (comprising memantine hydrochloride) and Comparative Example 21 (comprising memantine levulinate). In addition, Example 5 showed a constant skin permeation and release pattern for a long time compared to Comparative Example 19 (comprising memantine free base).

As shown in Figure 7, Example 6, which had oleoyl macrogol-6-glyceride added as a solubilizer, showed the same level of skin permeability as Example 1 and higher skin permeability than Comparative Example 23 (comprising memantine hydrochloride) and Comparative Example 24 (comprising memantine levulinate). In addition, Example 6 showed a constant skin permeation and release pattern for a long time compared to Comparative Example 22 (comprising memantine free base).

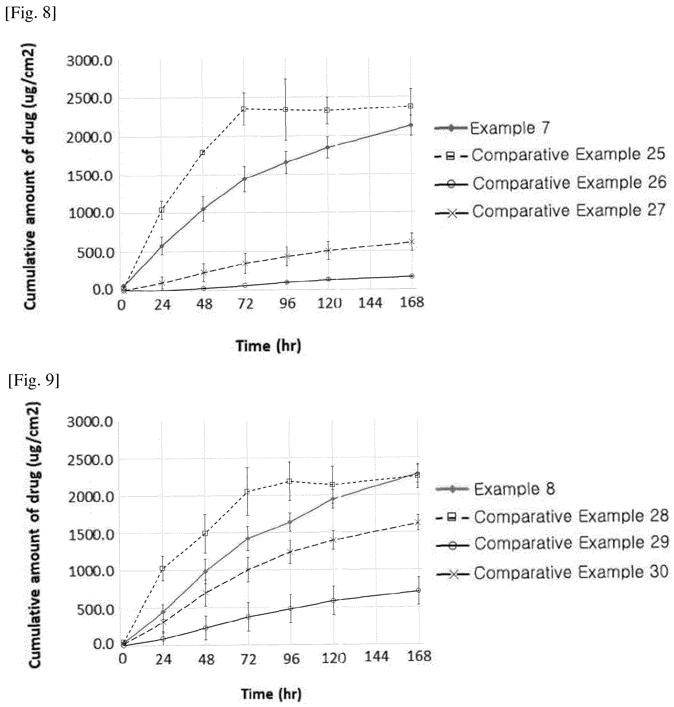

As shown in Figure 8, Example 7, which had isopropyl isostearate added as a solubilizer, showed the same level of skin permeability as Example 1 and higher skin permeability than Comparative Example 26 (comprising memantine hydrochloride) and Comparative Example 27 (comprising memantine levulinate). In addition, Example 7 showed a constant skin permeation and release pattern for a long time compared to Comparative Example 25 (comprising memantine free base).

As shown in Figure 9, Example 8, which had oleyl alcohol added as a solubilizer, showed the same level of skin permeability as Example 1 and higher skin permeability than Comparative Example 29 (comprising memantine hydrochloride) and Comparative Example 30 (comprising memantine levulinate). In addition, Example 8 showed a constant skin penetration and release pattern for a long time compared to Comparative Example 28 (comprising memantine free base).

### 3. Skin permeability evaluation of memantine enanthate percutaneous absorption preparation with reduced solubilizer content and different adhesive

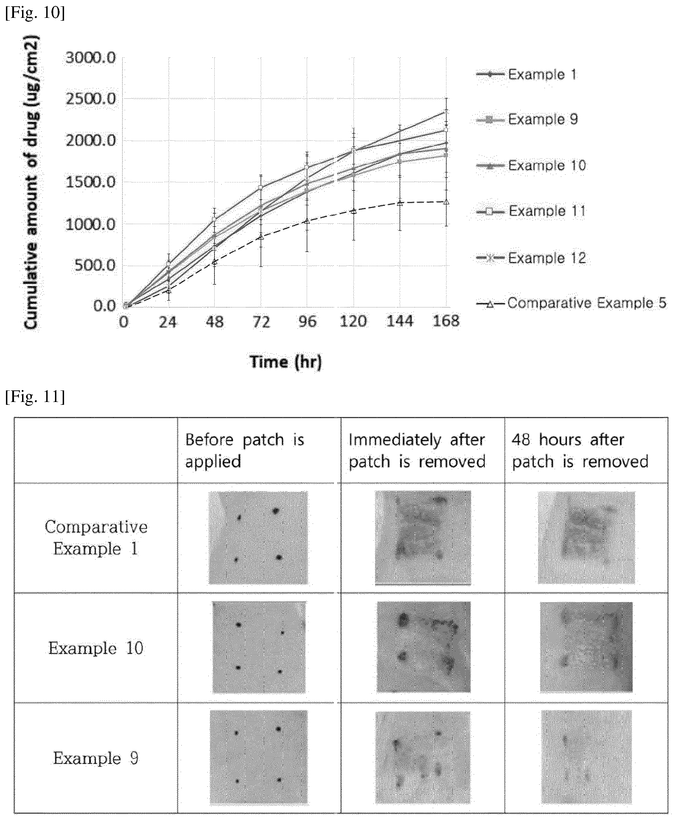

As shown in Figure 10, Examples 9 and 10, which were prepared by reducing the content of solubilizer of Examples 1 and 3 (propylene glycol monocaprylate and diisopropyl adipate, respectively), exhibited the equivalent level of skin permeability as Examples 1 and 3, unaffected by the amount of the solubilizer. Example 11, in which the pressure-sensitive adhesive composition of Example 3 was changed to an acrylic pressure-sensitive adhesive, also exhibited the same level of skin permeability as Examples 1 and 3, irrespective of the pressure-sensitive adhesive change. In addition, Example 12, which was identical to the prior art example in the composition except with the active ingredient switched to memantine enanthate, showed skin permeability that was equivalent to that of Examples 1 and 3, even though a polyisobutylene-based adhesive was used, and it also exhibited a skin permeability 1.85 times higher compared to Comparative Example 5 comprising memantine levulinate in the same composition. Through these results, it was confirmed that the percutaneous absorption preparation comprising memantine enanthate still exhibited skin permeability even when the solubilizer was reduced and various other adhesives were used.

### <Experimental Example 6> Drug crystal precipitation study

Drug crystal precipitation of the percutaneous absorption preparations prepared in Examples 1 through 10 and Comparative Example 2 were evaluated. The crystal precipitation pattern was observed under a microscope after storing the percutaneous absorption preparations prepared according to Examples 1 to 10 at 25°C and 60% RH for 1 month. The results are shown in Table 17 below.

**[Table 17]**

| | Example 1 | Example 2 | Example 3 | Example 4 | Example 5 | Example 6 | Example 7 | Example 8 | Example 9 | Example 10 | Comparative Example 2 |
|---|---|---|---|---|---|---|---|---|---|---|---|
| Crystal precipitation | X | X | X | X | X | X | X | X | X | X | 0 |

As shown in Table 17, Examples 1 through 8 with varying solubilizers did not show crystal formation, whereas Comparative Example 2 comprising memantine hydrochloride showed crystallization. Furthermore, no crystallization occurred even when memantine enanthate and the solubilizers of propylene glycol monocaprylate to diisopropyl adipate of Examples 9 and 10 were added at a 1:1 ratio.

### <Experimental Example 7> Skin irritation evaluation

The degree of skin irritation of the memantine percutaneous absorption preparation according to the present invention was evaluated. For this evaluation, Examples 9 and 10 comprising memantine enanthate of the present invention, Comparative Example 1 comprising the same dose of memantine free base, Comparative Example 1-1 comprising memantine free base content reduced to 70%, Comparative Examples 3 and 5 comprising the same amount of memantine levulinate, and Comparative Example 6 comprising memantine hydrochloride were evaluated for comparison.

### 1. Evaluation of skin irritation in rabbits

After removing the hair of 5 white rabbits, Examples 9 and 10, Comparative Examples 1-1, 3, 5, and 6 were cut by 5 cm2 and attached, and removed after 7 days. The formation of erythema and edema at the site where the percutaneous absorption preparation was attached was evaluated by observing the skin reaction with the naked eye immediately after removing the patch and 48 hours after removal, using the evaluation criteria described in Table 18, using the primary irritation index (PII) according to Table 19. The results are shown in Table 20 and Figure 11.

### 2. Evaluation of skin irritation in hairless mice

After removing the hair of 4 hairless mice, Examples 9 and 10, Comparative Examples 1, 3, 5, and 6 were cut by 1 cm2 and attached, and removed after 7 days. The formation of erythema and edema at the site where the percutaneous absorption preparation was attached was evaluated by observing the skin reaction with the naked eye immediately after removing the patch and 48 hours after removal, using the evaluation criteria described in Table 18, using the primary irritation index (PII) according to Table 19. The results are shown in Table 20.

**[Table 18]**

| Observation of skin reaction | | | |
|---|---|---|---|
| **Erythema and eschar formation** | | **Edema formation** | |
| | Score | | Score |
| No erythema | 0 | No edema | 0 |
| Very slight erythema (barely perceptible) | 1 | Very slight edema (barely perceptible) | 1 |
| Well-defined erythema | 2 | Slight edema (edges of area well-defined by definite raising) | 2 |
| Moderate to severe erythema | 3 | Moderate edema (raised approximately 1.0 mm) | 3 |
| Severe erythema (beet redness) to slight eschar formation (injuries in depth) | 4 | Severe edema (raised more than 1.0 mm beyond the area of exposure) | 4 |
| Maximum score | 4 | Maximum score | 4 |

**[Table 19]**

| Primary skin irritation index (P.I.I.) | |
|---|---|
| Score (P.I.I.) | Classification |
| 0.0 ~ 0.5 | Non-irritant |
| 0.6 ~ 2.0 | Slight irritant |
| 2.1 ~ 5.0 | Moderate irritant |
| 5.1 ~ 8.0 | Severe irritant |

**[Table 20]**

| | | **Example 9** | **Example 10** | **Comparative Example 1** | **Comparative Example 1-1** | **Comparative Example 3** | **Comparative Example 5** | **Comparative Example 6** |
|---|---|---|---|---|---|---|---|---|
| **Rabbit (7 days, n=5)** | PII | 0.6 | 1.5 | - | 2.1 | 0.2 | 0.9 | 1 |
| | Evaluation | Slight irritation | Slight irritation | - | Moderate irritation | No irritation | Slight irritation | Slight irritation |
| | Residual amount (%) | 19.1 | 1.9 | - | 0 | 54.8 | 31.8 | 72.9 |
| **Hairless mice (7 days, n=4)** | PII | 0.75 | 0.38 | 5.25 | - | 0.75 | 0.75 | 0.75 |
| | Evaluation | Slight irritation | No irritation | Severe irritation | - | Slight irritation | Slight irritation | Slight irritation |
| | Residual amount (%) | 15.2 | 17.4 | 1.5 | - | 66.5 | 76.7 | 78.9 |

As shown in Table 20 and Figure 11, from skin irritation evaluation in rabbits, Examples 9 and 10 comprising memantine enanthate of the present invention had skin irritation index scores of 0.6 and 1.5, respectively, which correspond to slight irritation. The residual amounts were 19.1% and 1.9%, respectively, indicating sufficient skin absorption of memantine. On the other hand, Comparative Example 1-1 comprising memantine free base had a skin irritation index score of 2.1, which correspond to moderate irritation with clear erythema and edema, despite the reduction in the memantine free base content, and there was no residual amount remaining. In addition, Comparative Examples 3 and 5 comprising memantine levulinate and Comparative Example 6 comprising memantine hydrochloride had a skin irritation index score of 1 or less, which correspond to no irritation or slight irritation, and the residual amount was 31.8-72.9%, indicating that their skin absorption level was lower than that of memantine enanthate.

As shown in Table 20, from skin irritation evaluation in hairless mice, Examples 9 and 10 comprising memantine enanthate of the present invention had skin irritation index scores of 0.75 and 0.38, respectively, which correspond to slight irritation or no irritation. The residual amounts were 15.2% and 17.4%, respectively, indicating sufficient skin absorption of memantine. On the other hand, Comparative Example 1 comprising memantine free base had a skin irritation index score of 5.25, corresponding to severe irritation due to severe erythema and eschar, and severe edema, and the residual amount was less than 1.5%. In addition, Comparative Examples 3 and 5 comprising memantine levulinate and Comparative Example 6 comprising memantine hydrochloride had a skin irritation index score of 1 or less, which correspond to no irritation or slight irritation, and the residual amount was 66.5-78.9%, indicating low skin absorption of memantine.

From this evaluation, the percutaneous absorption preparations comprising memantine enanthate of the present invention showed sufficient skin absorption compared to the percutaneous absorption preparations comprising memantine levulinate and memantine hydrochloride, and significantly lower skin irritation than the percutaneous absorption preparation comprising memantine free base.

### <Experimental Example 8> Pharmacokinetic evaluation

Pharmacokinetic evaluation of memantine percutaneous absorption preparation according to the present invention was performed. Comparative evaluation was performed for Example 10 comprising memantine enanthate of the present invention, Comparative Example 3 comprising memantine levulinate, and Reference Example 1 (Ebixa^{®}, an oral formulation comprising 10 mg of memantine hydrochloride, or 8.3 mg as free base).

### 1. Pharmacokinetic evaluation in rabbits

The percutaneous absorption preparation of Example 10 was molded to a size containing 106 mg and 160 mg of the free base, respectively, and Comparative Example 3, which is a percutaneous absorption preparation with an identical composition except for memantine levulinate, was molded to a size containing 140 mg of the free base. The patches were attached to the rabbit and removed 7 days later. Blood was collected at 0, 4, 8, 24, 30, 48, 72, 96, 144, and 168 hours after the patch was applied, to measure the amount of memantine in the blood plasma. In Reference Example 1, 1 tablet of Ebixa^{®} was suspended in 2 mL of 0.5% carboxymethylcellulose (CMC) solution, and 2 mL of the suspension was orally administered. Blood was collected at 0, 0.5, 1, 2, 4, 6, and 24 hours after the oral administration, to measure the amount of memantine in the blood plasma.

**[Table 21]**

| Pharmacokinetic evaluation in rabbits | | | | | |
|---|---|---|---|---|---|
| | **Reference Example 1 (Ebixa^{®} 10 mg)** | | **Example 10** | | **Comparative Example 3** |
| **Free base dose (mg/head)** | 8.3mg | | 106mg | 160 mg | 140 mg |
| **Tmax (hr)** | 0.6±0.3 | | 8.0±0.0 | 16.0±6.9 | 16.0±6.9 |
| **Cmax(ng/mL)** | 16.0±4.8 | | 318.1 ±98.9 | 456.0 ±111.0 | 41.8 ±3.63 |
| **AUC (ng*hr/mL)** | 1days =2AUC(20mg) | 148.8±61.9 | 10518.1 ±2661.4 | 18456.6 ±6031.8 | 3381.2 ±244.6 |
| **Normalized Cmax (%)** | 100 | | 155.7 | 147.8 | 15.5 |
| **Normalized AUC (%)** | 100 | | 553.5 | 643.4 | 134.7 |

As can be seen from Table 21 above, pharmacokinetic evaluation was performed after Example 10 comprising memantine enanthate of the present invention and Comparative Example 3 comprising memantine levulinate were applied for 7 days as a patch, and Reference Example 1, the Ebixa^{®} 10 mg tablet, was orally administered, to rabbits. The 106 mg patch of Example 10 had an AUC of 10518.1 ng*hr/mL, and the 140 mg patch of Comparative Example 3 had an AUC of 3381.2 ng*hr/mL. The AUC of one day dose oral administration of Reference Example 1 was 148.8 ng*hr/mL. After conversion to an equivalent dose of the drug, the AUC of Example 10 was about 550% improved compared to Reference Example 1, Comparative Example 3 was improved by 130%, indicating that the percutaneous absorption preparation comprising memantine enanthate according to the present invention achieved a significantly higher drug concentration than the oral tablet comprising memantine hydrochloride and Comparative Example 3 comprising memantine levulinate. Furthermore, evaluation following molding the size of the patch according to the dose of Example 10 found that the blood concentration increased when the area of the percutaneous absorption preparation was increased, indicating that the dose of administration can be adjusted easily by adjusting the patch size.

### 2. Pharmacokinetic evaluation in hairless mice

Example 10 was molded to a size containing 10 mg, 20 mg, and 30 mg as a free base dose, respectively, and Comparative Example 3 was molded to a size containing 9 mg as a free base dose, and Reference Example 1 was administered in the same manner as above 1. Pharmacokinetic evaluation in rabbits. Afterwards, blood was collected at 0, 0.5, 1, 2, 4, 6, and 24 hours after administration to measure the amount of memantine in plasma.

**[Table 22]**

| Pharmacokinetic evaluation in hairless mice | | | |
|---|---|---|---|
| | **Reference Example 1 (Ebixa^{®} 10 mg)** | **Example 10** | **Comparative Example 3** |
| **Free base dose (mg/head)** | Base 8.3mg | 10 mg | 9 mg |
| **Cmax (ng/mL)** | 410.3±67.6 | 105.5±23.2 | 60.7±16.6 |
| **AUClast(ng*h/mL)** | 2862.2±373.3 | 6374.7±1783.2 | 3999.0±407.4 |
| **Tmax (h)** | 0.9±0.8 | 10.2±11.1 | 15.0±10.4 |
| **T1/2 (h)** | 8.8±2.7 | 43.0±10.8 | 85.4±16.8 |
| **Normalized Cmax (%)** | 100 | 21.3 | 13.6 |
| **Normalized AUC (%)** | 100 | 184.9 | 128.9 |

As can be seen from Table 22 above, pharmacokinetic evaluation was performed after Example 10 comprising memantine enanthate of the present invention and Comparative Example 3 comprising memantine levulinate were applied for 7 days as a patch, and Reference Example 1, the Ebixa^{®} 10 mg tablet was orally administered, to hairless mice. The 10 mg patch of Example 10 had an AUC of 6374.7 ng*hr/mL, and the 9 mg patch of Comparative Example 3 had an AUC of 3999.0 ng*hr/mL. The AUC of one day dose oral administration of Reference Example 1 was 2862.2 ng*hr/mL. After conversion to an equivalent dose of the drug, the AUC of Example 10 and Comparative Example 3 were about 185% and 130% improved, respectively, compared to Reference Example 1, indicating that the percutaneous absorption preparation comprising memantine enanthate according to the present invention achieved higher drug concentration than the oral tablet comprising memantine hydrochloride and Comparative Example 3 comprising memantine levulinate.

**[Table 23]**

| Pharmacokinetic evaluation according to the increase in patch size in hairless mice | | | | |
|---|---|---|---|---|
| | **Reference Example 1 (Ebixa^{®} 10 mg)** | **Example 10** | | |
| **Free base dose (mg/head)** | Base 8.3mg | 10 mg | 20mg | 30 mg |
| **Cmax (ng/mL)** | 410.3±67.6 | 105.5±23.2 | 194.3±15.5 | 304.1±21.6 |
| **AUClast (ng*h/mL)** | 2862.2±373.3 | 6374.7±1783.2 | 11121.8±510.3 | 16491.4±1683.4 |
| **Tmax (h)** | 0.9±0.8 | 10.2±11.1 | 26.4±3.3 | 24.0±0.0 |
| **T1/2 (h)** | 8.8±2.7 | 43.0±10.8 | 30.4±12.8 | 27.4±5.8 |
| **Relative Cmax** (%) **vs. Oral** | 100 | 25.7 | 47.4 | 74.1 |
| **Relative AUC (%) vs. Oral** | 100 | 222.7 | 388.6 | 576.2 |

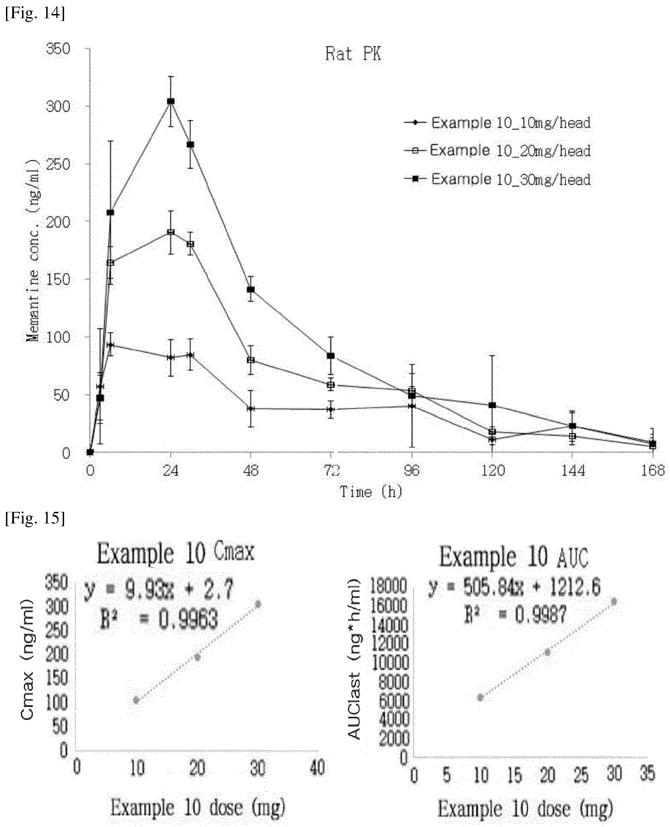

In addition, as can be seen from Table 23 and Figure 15, the evaluation following molding the size of the patch of Example 10 to contain a dose of 10 mg, 20 mg, and 30 mg, respectively, found that the maximum plasma concentration and area under the drug concentration level-time curve increased proportionally when the area of the percutaneous absorption preparation was increased, indicating that the dose of administration can be adjusted easily by adjusting the patch size.

As shown in the results above, the present invention provides a percutaneous absorption preparation comprising memantine enanthate, which does not form crystal precipitation, exhibits high skin permeability compared to the percutaneous absorption preparations comprising other salts of memantine, and exhibits dose-proportional systemic exposure allowing the dosage to be easily adjusted clinically by adjusting the patch size and has the benefit of reducing the area. Another advantage of the present invention is that skin irritation caused by drugs can be minimized by controlling the absorption rate of the skin by controlling the release rate. Furthermore, the residual amount of the drug in the patch after administration was shown to be about 20% by weight or less than the content of the drug before administration, providing the economic advantage of minimizing the loss of the remaining drug and the clinical advantage of preventing overdose of the drug at the same time.

## Claims

1. A memantine-containing percutaneous absorption preparation for the treatment of dementia comprising a support layer, a drug-containing layer, and a release layer, wherein the drug-containing layer comprises memantine enanthate, a solubilizer, and a pressure-sensitive adhesive.

2. The percutaneous absorption preparation for the treatment of dementia of claim 1, wherein the content of the memantine enanthate is 1 to 20% by weight of the total weight of the drug-containing layer.

3. The percutaneous absorption preparation for the treatment of dementia of claim 1, wherein the solubilizer has a solubility of 30 mg/ml or more in memantine enanthate.

4. The percutaneous absorption preparation for the treatment of dementia of claim 3, wherein the solubilizer is selected from the group consisting of propylene glycol monocaprylate, propylene glycol monolaurate, diisopropyl adipate, diisopropyl sebacate, oleic acid, isopropyl palmitate, glyceryl monocaprylate, isostearate, cocoyl caprylocaprate, cetyl 2-ethylhexanoate, oleoyl macrogol-6-glyceride, propylene glycol dicaprylocaprate, propylene glycol dicaprylocaprate, propylene glycol di-caprorate/dicaprate, glyceryl monooleate, 1-dodecyl-2-pyrrolidinone, linoleoyl macrogol-6 glyceride, polyglyceryl-3 dioleate, ethyl oleate, isopropyl isostearate, isopropyl myristate, medium chain triglyceride, isocetyl myristate, isostearyl alcohol, oleyl alcohol, peanut oil, and diethylene glycol monoethyl ether.

5. The percutaneous absorption preparation for the treatment of dementia of claim 4, wherein the solubilizer is selected from the group consisting of propylene glycol monocaprylate, diisopropyl adipate, diisopropyl sebacate, isopropyl palmitate, isopropyl myristate, oleoyl macrogol-6-glyceride, isopropyl isostearate, and oleyl alcohol.

6. The percutaneous absorption preparation for the treatment of dementia of claim 1, wherein the solubilizer is present in an amount of 1 to 40% by weight of the total weight of the drug-containing layer.

7. The percutaneous absorption preparation for the treatment of dementia of claim 1, wherein the pressure-sensitive adhesive is selected from a group consisting of a styrene-isoprene-styrene block copolymer (SIS), acrylic polymer, and polyisobutylene polymer.

8. The percutaneous absorption preparation for the treatment of dementia of claim 7, wherein the pressure-sensitive adhesive is a styrene-isoprene-styrene block copolymer.

9. The percutaneous absorption preparation for the treatment of dementia of claim 8, wherein the content of the styrene-isoprene-styrene block copolymer is 10 to 70% by weight of the total weight of the drug-containing layer.

10. The percutaneous absorption preparation for the treatment of dementia of claim 7, wherein the pressure-sensitive adhesive is an acrylic polymer.

11. The percutaneous absorption preparation for the treatment of dementia of claim 10, wherein the content of the acrylic polymer is 50 to 90% by weight of the total weight of the drug-containing layer.

12. The percutaneous absorption preparation for the treatment of dementia of claim 7, wherein the pressure-sensitive adhesive is a polyisobutylene polymer.

13. The percutaneous absorption preparation for the treatment of dementia of claim 12, wherein the content of the polyisobutylene polymer is 10 to 90% by weight of the total weight of the drug-containing layer.

14. The percutaneous absorption preparation for the treatment of dementia of claim 1, wherein the drug-containing layer further comprises a plasticizer.

15. A method for preparing a percutaneous absorption preparation for the treatment of dementia comprising memantine enanthate, wherein the method comprises:
(a) dissolving a solubilizer, a pressure-sensitive adhesive, and memantine enanthate in an organic solvent;
(b) applying the solution prepared in the above step (a) to the release layer and drying it to form a drug-containing layer; and
(c) laminating the drug-containing layer with a support layer.

16. The method according to claim 15, wherein the organic solvent is selected from the group consisting of ethyl acetate, toluene, hexane, 2-propanol, methanol, ethanol, methylene chloride, and tetrahydrofuran.

## Patentansprüche

1. Memantin-haltiges Präparat zur perkutanen Absorption zur Behandlung von Demenz, umfassend eine Trägerschicht, eine arzneimittelhaltige Schicht und eine Trennschicht, wobei die arzneimittelhaltige Schicht Memantin-Enanthat, einen Lösungsvermittler und einen Haftklebstoff umfasst.

2. Perkutanes Absorptionspräparat zur Behandlung von Demenz nach Anspruch 1, wobei der Gehalt an Memantin-Enanthat 1 bis 20 Gew.-% des Gesamtgewichts der arzneimittelhaltigen Schicht beträgt.

3. Perkutanes Absorptionspräparat zur Behandlung von Demenz nach Anspruch 1, wobei der Lösungsvermittler eine Löslichkeit von 30 mg/ml oder mehr in Memantin-Enanthat aufweist.

4. Perkutanes Absorptionspräparat zur Behandlung von Demenz nach Anspruch 3, wobei der Lösungsvermittler ausgewählt ist aus der Gruppe, bestehend aus Propylenglykolmonocaprylat, Propylenglykolmonolaurat, Diisopropyladipat, Diisopropylsebacat, Ölsäure, Isopropylpalmitat, Glycerylmonocaprylat, Isostearat, Cocoylcaprylocaprat, Cetyl-2-ethylhexanoat, Oleoylmacrogol-6-glycerid, Propylenglykoldicaprylocaprat, Propylenglykoldicaprylocaprat, Propylenglykoldicaprorat/Dicaprat, Glycerylmonooleat, 1-Dodecyl-2-pyrrolidinon, Linoleoyl-Macrogol-6-glycerid, Polyglyceryl-3-dioleat, Ethyloleat, Isopropylisostearat, Isopropylmyristat, mittelkettiges Triglycerid, Isocetylmyristat, Isostearylalkohol, Oleylalkohol, Erdnussöl und Diethylenglykolmonoethylether.

5. Perkutanes Absorptionspräparat zur Behandlung von Demenz nach Anspruch 4, wobei der Lösungsvermittler ausgewählt ist aus der Gruppe, bestehend aus Propylenglykolmonocaprylat, Diisopropyladipat, Diisopropylsebacat, Isopropylpalmitat, Isopropylmyristat, Oleoylmacrogol-6-glycerid, Isopropylisostearat und Oleylalkohol.

6. Perkutanes Absorptionspräparat zur Behandlung von Demenz nach Anspruch 1, wobei der Lösungsvermittler in einer Menge von 1 bis 40 Gew.-% des Gesamtgewichts der arzneimittelhaltigen Schicht vorliegt.

7. Perkutanes Absorptionspräparat zur Behandlung von Demenz nach Anspruch 1, wobei der Haftklebstoff ausgewählt ist aus einer Gruppe, bestehend aus einem Styrol-Isopren-Styrol-Blockcopolymer (SIS), einem Acrylpolymer und einem Polyisobutylenpolymer.

8. Perkutanes Absorptionspräparat zur Behandlung von Demenz nach Anspruch 7, wobei der Haftklebstoff ein Styrol-Isopren-Styrol-Blockcopolymer ist.

9. Perkutanes Absorptionspräparat zur Behandlung von Demenz nach Anspruch 8, wobei der Gehalt des Styrol-Isopren-Styrol-Blockcopolymers 10 bis 70 Gew.-% des Gesamtgewichts der arzneimittelhaltigen Schicht beträgt.

10. Perkutanes Absorptionspräparat zur Behandlung von Demenz nach Anspruch 7, wobei der Haftklebstoff ein Acrylpolymer ist.

11. Perkutanes Absorptionspräparat zur Behandlung von Demenz nach Anspruch 10, wobei der Gehalt des Acrylpolymers 50 bis 90 Gew.-% des Gesamtgewichts der arzneimittelhaltigen Schicht beträgt.

12. Perkutanes Absorptionspräparat zur Behandlung von Demenz nach Anspruch 7, wobei der Haftklebstoff ein Polyisobutylenpolymer ist.

13. Perkutanes Absorptionspräparat zur Behandlung von Demenz nach Anspruch 12, wobei der Gehalt des Polyisobutylenpolymers 10 bis 90 Gew.-% des Gesamtgewichts der arzneimittelhaltigen Schicht beträgt.

14. Perkutanes Absorptionspräparat zur Behandlung von Demenz nach Anspruch 1, wobei die arzneimittelhaltige Schicht ferner einen Weichmacher umfasst.

15. Verfahren zur Herstellung eines perkutanen Absorptionspräparats zur Behandlung von Demenz, das Memantin-Enanthat umfasst, wobei das Verfahren umfasst:
(a) Auflösen eines Lösungsvermittlers, eines Haftklebstoffs und von Memantin-Enanthat in einem organischen Lösungsmittel;
(b) Auftragen der in dem obigen Schritt (a) hergestellten Lösung auf die Trennschicht und Trocknen derselben, um eine arzneimittelhaltige Schicht zu bilden; und
(c) Laminieren der arzneimittelhaltigen Schicht mit einer Trägerschicht.

16. Verfahren nach Anspruch 15, wobei das organische Lösungsmittel ausgewählt ist aus der Gruppe, bestehend aus Ethylacetat, Toluol, Hexan, 2-Propanol, Methanol, Ethanol, Methylenchlorid und Tetrahydrofuran besteht.

## Revendications

1. Préparation à absorption percutanée contenant de la mémantine pour le traitement de la démence, comprenant une couche de support, une couche contenant le médicament et une couche de protection, dans laquelle la couche contenant le médicament comprend de l'énanthate de mémantine, un solubilisant et un adhésif sensible à la pression.

2. Préparation à absorption percutanée pour le traitement de la démence selon la revendication 1, dans laquelle la teneur en énanthate de mémantine est comprise entre 1 et 20% en poids du poids total de la couche contenant le médicament.

3. Préparation à absorption percutanée pour le traitement de la démence selon la revendication 1, dans laquelle le solubilisant a une solubilité de 30 mg/ml ou plus dans l'énanthate de mémantine.

4. Préparation à absorption percutanée pour le traitement de la démence selon la revendication 3, dans laquelle le solubilisant est choisi dans le groupe constitué par le monocaprylate de propylène glycol, le monolaurate de propylène glycol, l'adipate de diisopropyle, le sébacate de diisopropyle, l'acide oléique, le palmitate d'isopropyle, le monocaprylate de glycéryle, l'isostéarate, caprylocaprate de cocoyle, le 2-éthylhexanoate de cétyle, l'oléoyl macrogol-6-glycéride, le dicaprylocaprate de propylène glycol, le dicaprylocaprate de propylène glycol, le dicaprorate/dicaprate de propylène glycol, le monooléate de glycéryle, l'1-dodécyl-2-pyrrolidinone, le macrogol-6 glycéride linoléique, le polyglycéryl-3 dioléate, l'oléate d'éthyle, l'isopropyl isostéarate, le myristate d'isopropyle, le triglycéride à chaîne moyenne, le myristate d'isocétyle, l'alcool isostéarylique, l'alcool oléylique, l'huile d'arachide et l'éther monoéthylique de diéthylène glycol.

5. Préparation à absorption percutanée pour le traitement de la démence selon la revendication 4, dans laquelle le solubilisant est choisi parmi le groupe constitué par le monocaprylate de propylène glycol, l'adipate de diisopropyle, le sébacate de diisopropyle, le palmitate d'isopropyle, le myristate d'isopropyle, le glycéride d'oléoyle macrogol-6, l'isostéarate d'isopropyle et l'alcool oléylique.

6. Préparation à absorption percutanée pour le traitement de la démence selon la revendication 1, dans laquelle le solubilisant est présent en une quantité de 1 à 40% en poids du poids total de la couche contenant le médicament.

7. Préparation à absorption percutanée pour le traitement de la démence selon la revendication 1, dans laquelle l'adhésif sensible à la pression est choisi parmi un groupe comprenant un copolymère séquencé styrène-isoprène-styrène (SIS), un polymère acrylique et un polymère polyisobutylène.

8. Préparation à absorption percutanée pour le traitement de la démence selon la revendication 7, dans laquelle l'adhésif sensible à la pression est un copolymère séquencé styrène-isoprène-styrène.

9. Préparation à absorption percutanée pour le traitement de la démence selon la revendication 8, dans laquelle la teneur en copolymère séquencé styrène-isoprène-styrène est comprise entre 10 et 70% en poids du poids total de la couche contenant le médicament.

10. Préparation à absorption percutanée pour le traitement de la démence selon la revendication 7, dans laquelle l'adhésif sensible à la pression est un polymère acrylique.

11. Préparation à absorption percutanée pour le traitement de la démence selon la revendication 10, dans laquelle la teneur en polymère acrylique est comprise entre 50 et 90% en poids du poids total de la couche contenant le médicament.

12. Préparation à absorption percutanée pour le traitement de la démence selon la revendication 7, dans laquelle l'adhésif sensible à la pression est un polymère de polyisobutylène.

13. Préparation à absorption percutanée pour le traitement de la démence selon la revendication 12, dans laquelle la teneur en polymère de polyisobutylène est comprise entre 10 et 90% en poids du poids total de la couche contenant le médicament.

14. Préparation à absorption percutanée pour le traitement de la démence selon la revendication 1, dans laquelle la couche contenant le médicament comprend en outre un plastifiant.

15. Procédé de préparation d'une préparation à absorption percutanée pour le traitement de la démence comprenant de l'énanthate de mémantine, dans lequel le procédé comprend :
(a) la dissolution d'un solubilisant, d'un adhésif sensible à la pression et d'énanthate de mémantine dans un solvant organique ;
(b) l'application de la solution préparée à l'étape (a) ci-dessus sur la couche de protection et son séchage pour former une couche contenant le médicament ; et
(c) laminer la couche contenant le médicament avec une couche de support.

16. Procédé selon la revendication 15, dans lequel le solvant organique est choisi dans le groupe constitué par l'acétate d'éthyle, le toluène, l'hexane, le 2-propanol, le méthanol, l'éthanol, le chlorure de méthylène et le tétrahydrofurane.
